# EUROPÄISCHE PATENTANMELDUNG

(11) **EP 2 441 484 A1**
(43) Veröffentlichungstag der Anmeldung: **18.04.2012**
(21) Anmeldenummer: 10187378.4
(22) Anmeldetag: 13.10.2010
(51) Int. Cl.: A61M 5/14, A61M 5/142, F04B 43/14, F04B 43/12, A61M 39/24, A61M 39/26, A61M 5/168

(54) **Pumpenmodul, Pumpenbasismodul und Pumpenssystem**

(71) Anmelder: Fresenius Kabi Deutschland GmbH, 61352 Bad Homburg (DE)
(72) Erfinder: Dr. Becker, Michael, 75438 Knittlingen (DE)
(74) Vertreter: Richly, Erik

(57) **Zusammenfassung**

Die Erfindung betrifft ein Pumpenmodul, ein Pumpenbasismodul und ein Pumpensystem, das ein Pumpenmodul und ein Pumpenbasismodul umfasst.

Das erfindungsgemäße Pumpmodul (1) umfasst einen linienförmigen, zumindest abschnittsweise gekrümmten Pumpkanal (5), einen Pumpkanaleingang (6) und einen Pumpkanalausgang (7), wobei der Pumpkanaleingang (6) und der Pumpkanalausgang (7) mit dem Pumpkanal (5) zum Zuführen und Abführen eines Fluids in den Pumpkanal (5) verbunden ist, so dass durch eine periodisch umlaufende Verformung des Pumpkanals (5) ein Fluid durch den Pumpkanal (5) vom Pumpkanaleingang (6) zum Pumpkanalausgang (7) pumpbar ist, wobei der Pumpkanal (5) in dem Abschnitt zwischen Pumpkanaleingang (6) und Pumpkanalausgang (7) abweichend von einer reinen Kreisbogenform ausgebildet ist.

## Beschreibung

### Technisches Gebiet

Die Erfindung betrifft ein Pumpenmodul, ein Pumpenbasismodul und ein Pumpensystem, das ein Pumpenmodul und ein Pumpenbasismodul umfasst. Insbesondere betrifft die Erfindung ein Pumpenmodul für eine Taumelpumpe, ein Taumelpumpenbasismodul und ein Taumelpumpensystem.

### Stand der Technik

Die US 5 466 133 offenbart eine Taumelpumpe. Die Taumelpumpe umfasst einen Pumpenantrieb und eine Taumelscheibe. Des Weiteren umfasst die Taumelpumpe eine Membran und ein unteres Gehäuseteil, die gemeinsam einen Pumpkanal ausbilden. Der Pumpkanal wird durch eine kreisbogenförmige Aussparung in dem Gehäuseteil gebildet, wobei der Kreis nicht geschlossen ist. Am einen Ende der kreisförmigen Aussparung befindet sich ein Pumpkanaleingang, an dem anderen Ende ein Pumpkanalausgang. Das untere Gehäuseteil ist über Schraubverbindungen fest mit einem mittleren Gehäuseteil verbunden. In dem mittleren Gehäuseteil ist die Taumelvorrichtung angeordnet. Die Membran ist zwischen unterem Gehäuseteil und mittlerem Gehäuseteil eingeklemmt. Des Weiteren ist die Membran fest mit der Taumelvorrichtung verbunden. Durch den Taumelantrieb kann die Taumelvorrichtung in eine taumelnde Bewegung versetzt werden, die durch die feste Verbindung mit der Membran an die Membran übertragen wird. Die taumelnde Bewegung der Membran entspricht einer peristaltischen Bewegung. Der Pumpkanal wird durch Verformung der Membran in einem Abschnitt zwischen Pumpeingang und Pumpausgang geschlossen, und der geschlossene Abschnitt läuft mit der taumelnden Bewegung der Taumelvorrichtung von Pumpeingang zu Pumpausgang mit. Auf diese Weise ist ein Fluid durch den Pumpkanal förderbar. Ein ungewünschter Rückfluss wird durch Rückschlagventile hinter dem Pumpkanaleingang und dem Pumpkanalausgang verhindert.

Die DE 32 27 051 A1 offenbart eine Schlauchpumpe für medizinische Anwendungen. Die Schlauchpumpe ist als Taumelpumpe ausgebildet. Die Schlauchpumpe umfasst einen Pumpenantrieb und eine Taumelscheibe, die mittels des Pumpenantriebs in eine taumelnde Bewegung versetzbar ist. Die Schlauchpumpe weist einen aufklappbaren Deckel auf, in den ein Schlauch einlegbar ist. Der eingelegte Schlauch wird in einem kreisbogenförmigen Pumpabschnitt durch die taumelnde Taumelscheibe an einer mit der Taumelbewegung mitwandernden Stelle abgequetscht, so dass ein Fluid durch den Schlauch förderbar ist. Die Quetschstelle ist genügend lang, dass in einer Phase der Taumelbewegung ein Einlassabschnitt und ein Auslassabschnitt des Schlauches gemeinsam abgequetscht werden, so dass der Schlauch immer an zumindest einer Stelle geschlossen ist. Auf diese Weise wird ein ungewünschter Durchfluss des Fluids verhindert.

### Kurzbeschreibung der Erfindung

Das erfindungsgemäße Pumpenmodul für eine Taumelpumpe umfasst einen linienförmigen, zumindest abschnittsweise gekrümmten Pumpkanal, einen Pumpkanaleingang und einen Pumpkanalausgang, wobei der Pumpkanaleingang und der Pumpkanalausgang mit dem Pumpkanal zum Zuführen und Abführen eines Fluids in den Pumpkanal verbunden ist, so dass durch eine periodisch umlaufende Verformung des Pumpkanals ein Fluid durch den Pumpkanal vom Pumpkanaleingang zum Pumpkanalausgang pumpbar ist, wobei der Pumpkanal in dem Abschnitt zwischen Pumpkanaleingang und Pumpkanalausgang abweichend von einer reinen Kreisbogenform ausgebildet ist.

Unter einer "Kreisbogenform" ist im Rahmen der Erfindung ein kreisförmiger Bogen zu verstehen, der offen oder auch geschlossen sein kann (im letzteren Falle also einen Kreis bildet). Ein Pumpkanal mit kreisbogenförmigen Abschnitten, die aber mit unterschiedlichen Radien bezüglich eines gemeinsamen Zentrums ausgebildet sind, wird im Rahmen der Erfindung ebenfalls als von einer Kreisbogenform abweichend angesehen.

Die von einer reiner Kreisbogenform abweichende Ausbildung des Pumpkanals ermöglicht es beispielsweise, den Pumpkanaleingang und den Pumpkanalausgang im Rahmen der Taumelbewegung einer Taumelvorrichtung sicher zum selben Zeitpunkt zu verschließen, so dass verhindert wird, dass es einen Zustand gibt, in dem der Pumpkanal zwischen Pumpkanalausgang und Pumpkanaleingang geöffnet ist und ein ungewünschter Durchfluss des Fluids erfolgt. Alternativ oder zusätzlich ist es möglich, im Falle einer axial beweglich gelagerten Taumelvorrichtung und/oder zumindest teilweise nachfedernd ausgebildeten Taumelvorrichtung die axiale Amplitudenbewegung der Taumelvorrichtung oder Teile von dieser während ihrer periodisch umlaufenden Taumelbewegung zu optimieren, insbesondere in den Übergangsphasen, in denen die Taumelvorrichtung den pumpkanalfreien Abschnitt zwischen Pumpkanaleingang und Pumpkanalausgang überbrücken muss.

Des Weiteren kann alternativ oder zusätzlich durch die Ausbildung von sich überlappenden Pumpkanalabschnitten eine Kompression und damit eine Druckerhöhung eines durch den Pumpkanal geförderten Fluids erzielt werden. Alternativ oder zusätzlich ermöglicht die Abweichung des Pumpkanals von einer reinen Kreisbogenform, beispielsweise durch Aufspaltung des Pumpkanals in zumindest einen ersten Bereich und einen zweiten Bereich, einen Abschnitt des Pumpkanals für Messzwecke oder zum Zweck des Druckausgleiches vorzusehen.

Gemäß einer vorteilhaften Weiterbildung weist der Pumpkanal (5) in dem Abschnitt zwischen Pumpkanaleingang (6) und Pumpkanalausgang (7) einen kreisförmigen Abschnitt, einen ersten geraden Abschnitt und einen zweiten geraden Abschnitt auf, wobei der kreisförmige Abschnitt zwischen dem ersten geraden Abschnitt und dem zweiten geraden Abschnitt angeordnet ist.

Gemäß einer vorteilhaften Weiterbildung umfasst das Pumpmodul einen Schlauch, wobei ein Abschnitt des Schlauches den Pumpkanal ausbildet.

Gemäß einer vorteilhaften Weiterbildung umfasst das Pumpmodul ein Basisteil und eine elastisch verformbare Membran, wobei Basisteil und Membran den Pumpkanal ausbilden, und das Basisteil den Pumpkanaleingang und den Pumpkanalausgang umfasst.

Gemäß einer vorteilhaften Weiterbildung umfasst das Pumpmodul zusätzlich ein Deckelteil, wobei die Membran mit dem Deckelteil verbunden ist, und das Deckelteil mit dem Basisteil verbunden ist.

Besonders bevorzugt ist, dass die Membran mit dem Deckelteil stoffschlüssig verbunden ist, vorzugsweise an das Deckelteil angegossen oder ist. Insbesondere ist bevorzugt, dass Deckelteil und Membran ein einteiliges Zweikomponenten-Spritzgussteil ist.

Das erfindungsgemäße Taumelpumpenbasismodul umfasst einen Taumelpumpenantrieb mit einer Taumelvorrichtung, wobei die Taumelvorrichtung einen in eine Taumelbewegung versetzbaren, linienförmigen und zumindest abschnittsweise gekrümmten Steg zum taumelnden Verformen eines Schlauches oder einer Membran aufweist, wobei der Steg abweichend von einer reinen Kreisbogenform ausgebildet ist.

Dadurch, dass der Steg der Taumelvorrichtung abweichend von einer reinen Kreisbogenform ausgebildet ist, ist es möglich, den Pumpkanaleingang und den Pumpkanalausgang im Rahmen der Taumelbewegung einer Taumelvorrichtung sicher zum selben Zeitpunkt zu verschließen, so dass verhindert wird, dass es einen Zustand gibt, in dem der Pumpkanal zwischen Pumpkanalausgang und Pumpkanaleingang für einen Moment geöffnet ist und ein ungewünschter Durchfluss des Fluids erfolgt. Des Alternativ oder zusätzlich ermöglicht ein von einer Kreisbogenform abweichender Steg in Zusammenwirkung mit einem entsprechend ausgebildeten Abschnitt des Pumpkanals im Rahmen der Taumelbewegung eine Druckerhöhung innerhalb eines Bereiches des Pumpkanals zu erzeugen.

Gemäß einer vorteilhaften Weiterbildung ist der Steg in einem Abschnitt ausgespart oder weist eine Lücke auf.

Alternativ kann, anstatt die Taumelvorrichtung mit einem Steg auszubilden, die Membran mit einem entsprechenden Wulst ausgebildet sein. Die Taumelvorrichtung kann in diesem Falle eine plane Fläche aufweisen, die mit dem Wulst der Membran zusammenwirkt.

Das erfindungsgemäße Taumelpumpensystem umfasst ein erfindungsgemäßes Pumpenmodul und ein erfindungsgemäßes Taumelpumpenbasismodul, wobei der Steg der Taumelvorrichtung des Taumelpumpenbasismoduls in seinem Verlauf zumindest abschnittsweise entsprechend dem Abschnitt des Pumpkanals zwischen Pumpkanaleingang und Pumpkanalausgang ausgebildet ist.

Steg der Taumelvorrichtung und Pumpkanal korrespondieren zumindest abschnittsweise in ihrer von einem reinen Kreisbogen abweichenden Form. Der Steg kann in einer oder beiden Richtungen fortgesetzt sein, so dass der mit dem Pumpkanal korrespondierende Teil des Steges nur einen Teilabschnitt des Steges bildet.

Ein weiteres erfindungsgemäßes Pumpenmodul umfasst ein Basisteil und eine elastisch verformbare Membran, wobei Basisteil und Membran einen linienförmigen, zumindest abschnittsweise gekrümmten Pumpkanal ausbilden, und das Basisteil ein Pumpkanaleingang und einen Pumpkanalausgang aufweist, wobei der Pumpkanaleingang und der Pumpkanalausgang mit dem Pumpkanal zum Zuführen und Abführen eines Fluids in den Pumpkanal verbunden ist, derart, dass durch eine periodisch umlaufende Verformung der Membran ein Fluid durch den Pumpkanal vom Pumpkanaleingang zum Pumpkanalausgang pumpbar ist.

Das erfindungsgemäße Pumpmodul kann preiswert und robust hergestellt werden. Durch die Ausbildung des Pumpkanals aus Membran und Basisteil kann ein Pumpkanal mit definierten und reproduzierbaren Abmessungen erzeugt werden, wodurch für eine Vielzahl von Pumpmodulen eine hohe Genauigkeit in der Förderrate ermöglicht ist. Dadurch, dass das Pumpmodul kostengünstig und reproduzierbar herstellbar ist, eignet sich das erfindungsgemäße Pumpmodul als Einwegartikel ("Disposable"), der lediglich zum einmaligen Gebrauch vorgesehen ist.

Das Pumpmodul ist insbesondere als Bestandteil einer Taumelpumpe einsetzbar. Grundsätzlich kann ein erfindungsgemäßes Pumpmodul auch für andere Pumpentypen verwendbar sein.

Ein weiteres erfindungsgemäßes Taumelpumpenbasismodul umfasst einen Taumelpumpenantrieb mit einer Taumelvorrichtung und eine Aufnahme, wobei die Aufnahme derart ausgebildet ist, dass in die Aufnahme das Pumpenmodul händisch eingesetzt oder eingelegt und aus der Aufnahme das Pumpenmodul händisch entnommen werden kann.

Das erfindungsgemäße Taumelpumpenbasismodul ermöglicht es, in diesem händisch ohne die Zuhilfenahme von zusätzlichem Werkzeug ein Pumpenmodul einzusetzen und auf diese Weise eine funktionierende Taumelpumpe herzustellen, und ebenso einfach das Pumpenmodul dem Taumelpumpenbasismodul zu entnehmen. Das Taumelpumpenbasismodul ist aufgrund dessen insbesondere für den Einsatz von Pumpenmodulen geeignet, die als Einmalartikel ("Disposable") ausgebildet sind, und die üblicherweise nach jedem Gebrauch ersetzt werden.

Gemäß einer vorteilhaften Weiterbildung ist die Taumelvorrichtung des Taumelpumpenbasismoduls entlang ihrer Drehachse axial beweglich gelagert. Die axiale Lagerung der Taumelvorrichtung ermöglicht es, dass sich die Taumelvorrichtung relativ zum Pumpenbasismodul verschieben kann, um einen definierten Anpressdruck auf die Membran des Pumpenmoduls auszuüben.

Gemäß einer weiteren vorteilhaften Weiterbildung umfasst die Taumelvorrichtung Druckmessmittel, die es ermöglichen, den Druck innerhalb des Pumpkanals durch die Membran des Pumpenbasismoduls zu messen. Durch die Messung des Druckes im Pumpkanal kann, je nach Bewegungszustand der Taumelvorrichtung, der Druck bei geschlossenem Pumpkanalein- und -ausgang, bei offenem Pumpkanaleingang und bei offenem Pumpkanalausgang gemessen werden.

Ein weiteres erfindungsgemäßes Taumelpumpensystem umfasst ein erfindungsgemäßes Pumpenmodul und ein erfindungsgemäßes Taumelpumpenbasismodul, wobei das Pumpenmodul in der Aufnahme des Taumelpumpenbasismoduls aufgenommen ist.

Ein weiteres erfindungsgemäßes Pumpmodul für eine medizinische Pumpe, insbesondere für eine Taumelpumpe, umfasst einen Pumpkanal und eine mit dem Pumpkanal verbundene Ventilkammer, wobei durch Pumpkanal und Ventilkammer ein Fluid pumpbar ist, wobei ein erster Wandabschnitt der Ventilkammer flexibel ist, und das Pumpenmodul einen beweglichen Ventilkörper aufweist, der in der Ventilkammer angeordnet ist, wobei der Ventilkörper eine Ruhestellung einnehmen kann, in der der Ventilkörper die Ventilkammer gegen ein Durchtritt des Fluids verschließt, und eine Betätigungsstellung einnehmen kann, in der der Ventilkörper einen Durchfluss des Fluids durch die Ventilkammer freigibt, und über eine Verformung des ersten Wandabschnitts der Ventilkörper in die Betätigungsstellung bringbar ist, um den Durchfluss des Fluids durch die Ventilkammer zu ermöglichen.

Mittels des Ventilkörpers kann ein ungewollter Durchfluss des Fluids durch das Pumpmodul verhindert werden. Das auf diese Weise gebildete Ventil wird vorzugsweise als "Anti-Free-Flow-Ventil" verwendet, also als Ventil, das in der Grundstellung geschlossen ist und auf diese Weise verhindert, dass ein Fluid ungewünscht durch das Pumpmodul hindurchfließt. Dies betrifft insbesondere den Zustand, in dem der Pumpkanal noch offen ist, beispielsweise weil das Pumpmodul noch nicht in einem zugehörigen Pumpenbasismodul eingesetzt ist. Erst durch aktives Öffnen des Ventils wird ein Durchfluss durch das Pumpmodul freigegeben. Das Öffnen des Ventils erfolgt über die Verformung des ersten Wandabschnittes. Beispielsweise wird mittels der Verformung des Wandabschnitts der Ventilkörper in die Bestätigungsstellung gedrückt oder alternativ ein Raum freigegeben, der durch den Ventilkörper eingenommen werden kann. Dadurch, dass die Betätigung des Ventilkörpers über einen flexiblen Wandabschnitt erfolgt, sind die Mittel zur Betätigung des flexiblen Wandabschnitts, sei es beispielsweise eine Hand oder ein mechanisches Mittel, nicht in direktem Kontakt mit einem von dem Pumpenmodul förderbaren Fluid. Zu dem ist ein Pumpenmodul mit einem derartigen Ventil kostengünstig herstellbar, wodurch sich das Pumpenmodul insbesondere als Einwegartilkel ("Disposable") eignet.

Besonders bevorzugt ist, dass das Pumpenmodul als Modul für eine Taumelpumpe ausgebildet ist und ein Basisteil und einen Membran aufweist, die einen Pumpkanal ausbilden. Das Basisteil kann zumindest einen Abschnitt der Ventilkammer ausbilden. Dies ermöglicht es, mit wenigen Teilen ein Pumpenmodul herzustellen, welches kostengünstig und robust ist, und eine ausreichende Genauigkeit, insbesondere für medizinische Anwendungen, aufweist.

Ein weiteres erfindungsgemäßes Pumpenbasismodul umfasst einen Pumpenantrieb, eine Aufnahme und eine Ventilbetätigungsvorrichtung, wobei die Aufnahme derart ausgebildet ist, dass in die Aufnahme das Pumpenmodul eingesetzt oder eingelegt und aus der Aufnahme das Pumpenmodul entnommen werden kann, wobei die Ventilbetätigungsvorrichtung derart ausgebildet ist, dass diese beim Einsetzen oder Einlegen des Pumpenmoduls oder nach dem Einsetzen oder Einlegen des Pumpmoduls den ersten flexiblen Wandabschnitt der Ventilkammer des Pumpmoduls verformt und damit den Ventilkörper in die Betätigungsstellung bringt.

Das erfindungsgemäße Pumpenbasismodul bringt den Ventilkörper in die Betätigungsstellung, öffnet also das Ventil des Pumpenmoduls. Damit kann gewährleistet werden, dass das Ventil erst dann geöffnet wird, wenn es erwünscht ist, beispielsweise das Pumpenmodul korrekt eingelegt ist, und möglicherweise auch das Pumpenbasismodul einen bestimmten Zustand eingenommen hat, beispielsweise nach dem Starten eines Initialisierungs-oder Pumpprogramms.

Ein weiteres erfindungsgemäßes Pumpsystem umfasst ein erfindungsgemäßes Pumpmodul und ein erfindungsgemäßes Pumpenbasismodul.

Ein weiteres erfindungsgemäßes Taumelpumpenbasismodul umfasst einen Taumelpumpenantrieb, eine Taumelvorrichtung, eine Aufnahme für ein Pumpenmodul und eine Vorspanneinrichtung, wobei die Vorspanneinrichtung das in der Aufnahme aufgenommene Pumpenmodul gegen die Taumelvorrichtung federnd vorspannt.

Durch die federnde Vorspannung des Pumpenmoduls gegen die Taumelvorrichtung kann gewährleistet werden, dass das aufgenommene Pumpmodul und die Taumelvorrichtung eine definierte Position relativ zueinander einnehmen. Dies ist insbesondere für den Fall vorteilhaft, dass das Pumpenmodul als Einwegartikel ("Disposable") ausgebildet ist, also nach einmaligem Gebrauch entfernt und durch ein neues Pumpmodul ersetzt wird. Durch die definierten Positionen von Taumelvorrichtung und Pumpmodul kann verhindert werden, dass sich die Pumpeigenschaften beim Wechsel des Pumpmoduls in einem ungewünschten Ausmaße verändern.

Ein weiteres erfindungsgemäße Taumelpumpensystem umfasst ein erfindungsgemäßes Taumelpumpenbasismodul und ein Pumpenmodul, wobei das Pumpenmodul ein Basisteil und eine elastisch verformbare Membran umfasst, wobei Basisteil und Membran einen linienförmigen, zumindest abschnittsweise gekrümmten Pumpkanal ausbilden derart, dass durch eine taumelnde Verformung der Membran ein Fluid durch den Pumpkanal pumpbar ist, wobei das Pumpenmodul in der Aufnahme des Taumelpumpenbasismoduls derart aufgenommen ist, dass das Pumpmodul und Taumelvorrichtung durch die Vorspanneinrichtung federnd gegeneinander gepresst werden.

Die abhängigen Ansprüche beschreiben weitere bevorzugte Ausführungsformen der Erfindung.

Die hier beschriebenen Pumpmodule, Pumpenbasismodule, insbesondere Taumelpumpenbasismodule, Pumpsysteme, insbesondere Taumelpumpensysteme, eignen sich für die Anwendung auf dem medizinischen Gebiet. Bevorzugte Anwendungen dieser Vorrichtungen sind die Anwendung als enterale Pumpen zum Pumpen von beispielsweise Ernährungslösungen oder als Infusionspumpen zum intravenösen Infundieren von Medikamenten. Weitere Anwendungen, wie epidurale Infusionen, intramuskuläre oder subkutane Infusionen sind ebenfalls denkbar.

Die Erfindung wird im Folgenden anhand von Ausführungsbeispielen näher erläutert. Die Ausführungsbeispiele sind durch mehrere Figuren dargestellt.

### Kurze Beschreibung der Zeichnungsfiguren

Die Figuren zeigen:

- Fig. 1: eine Draufsicht auf ein Deckelteil mit Membran einer ersten Ausführungsform eines Pumpmoduls,

- Fig. 2: eine Draufsicht auf ein Basisteil der ersten Ausführungsform des Pumpmoduls,

- Fig. 3: eine Taumelvorrichtung in einer perspektivischen Ansicht,

- Fig. 4: einen Schnitt durch die Taumelvorrichtung und das Pumpmodul gemäß der ersten Ausführungsform entlang einer Schnittlinie A-A, siehe Fig. 1,

- Fig. 5: einen Schnitt durch die Taumelvorrichtung und das Pumpmodul gemäß der ersten Ausführungsform entlang der Schnittlinie B-B, siehe Fig. 1, wobei die Taumelvorrichtung sich in einem Zustand befindet, in dem Pumpkanalausgang und Pumpkanaleingang verschlossen sind,

- Fig. 6: einen Schnitt durch eine zweite Ausführungsform eines Pumpmoduls, wobei die Elemente des Pumpmoduls in einer Explosionsansicht dargestellt sind,

- Fig. 7: die zweite Ausführungsform des Pumpmoduls im zusammengesetzten Zustand,

- Fig. 8: eine Draufsicht auf ein Deckelteil und Membran einer dritten Ausführungsform eines Pumpmoduls,

- Fig. 9: eine perspektivische Ansicht einer zweiten Ausführungsform einer Taumelvorrichtung,

- Fig. 10: einen Schnitt durch die dritte Ausführungsform des Pumpenmoduls und der Taumelvorrichtung entlang der in Fig. 8 eingezeichneten Schnittlinie A-A,

- Fig. 11: eine Draufsicht auf ein Basisteil einer vierten Ausführungsform eines Pumpenmoduls,

- Fig. 12: einen Schnitt durch die vierte Ausführungsform des Pumpenmoduls entlang der in Fig. 11 eingezeichneten Schnittlinie A-A,

- Fig. 13: einen Schnitt durch die vierte Ausführungsform des Pumpenmoduls und der Taumelvorrichtung entlang der in Fig. 11 eingezeichneten Schnittlinie A-A,

- Fig. 14: eine Draufsicht auf ein Basisteil einer fünften Ausführungsform eines Pumpmoduls,

- Fig. 15: eine Draufsicht auf ein Deckelteil und Membran der fünften Ausführungsform eines Pumpmoduls,

- Fig. 16: eine Draufsicht auf ein Deckelteil und Membran einer sechsten Ausführungsform eines Pumpmoduls,

- Fig. 17: eine Draufsicht auf ein Basisteil der sechsten Ausführungsform eines Pumpmoduls,

- Fig. 18: eine Unteransicht auf eine Taumelvorrichtung gemäß einer dritten Ausführungsform,

- Fig. 19: eine Seitenansicht der in Fig. 18 gezeigten Taumelvorrichtung,

- Fig. 20: eine Seitenansicht einer Variante der in Fig. 18 gezeigten Taumelvorrichtung,

- Fig. 21: eine Aufsicht auf eine siebte Ausführungsform eines Pumpenmoduls,

- Fig. 22: einen Schnitt durch das in Fig. 21 gezeigte Pumpenmodul entlang der Schnittlinie C-C mit teilweise abgequetschtem Pumpkanal,

- Fig. 23: einen Schnitt durch das in Fig. 21 gezeigte Pumpenmodul entlang der Schnittlinie D-D,

- Fig. 24: einen Schnitt durch das Pumpmodul gemäß erster Ausführungsform entlang der Schnittlinie E-E, siehe Fig. 1, wobei sich ein Ventilkörper des Pumpmoduls in einer Ruhestellung befindet,

- Fig. 25: einen Schnitt durch das Pumpmodul gemäß erster Ausführungsform entlang der Schnittlinie E-E, siehe Fig. 1, wobei sich der Ventilkörper des Pumpmoduls in einer Betätigungsstellung befindet,

- Fig. 26: einen Schnitt durch eine achte Ausführungsform eines Pumpmoduls entlang der Schnittlinie E-E, siehe Fig. 1, wobei sich ein Ventilkörper des Pumpmoduls in einer Ruhestellung befindet,

- Fig. 27: einen Schnitt durch das achte Pumpmodul entlang der Schnittlinie E-E, siehe Fig. 1, wobei sich der Ventilkörper des Pumpmoduls in einer Betätigungsstellung befindet,

- Fig. 28: eine Variante des in Fig. 8 gezeigten Deckelteil, bei der der Wulst der Membran in einem Abschnitt unterbrochen ist,

- Fig. 29: einen Schnitt durch ein Pumpmodul mit dem in Fig. 28 gezeigten Deckelteil und einer Taumelvorrichtung entlang der Schnittlinie F-F ,

- Fig. 30: eine Draufsicht auf ein Taumelpumpenbasismodul mit einem geöffnetem Deckel und einer Aufnahme für ein Pumpenmodul,

- Fig. 31: eine Draufsicht auf das in Fig. 30 gezeigte Taumelpumpenbasismodul mit einem in der Aufnahme aufgenommenen Pumpenmodul,

- Fig. 32: einen Schnitt durch das Taumelpumpenbasismodul mit einem in der Aufnahme aufgenommenen Pumpenmodul bei geschlossenem Deckel,

- Fig. 33: einen Schnitt durch das Taumelpumpenbasismodul mit einem in der Aufnahme aufgenommenen Pumpenmodul bei geschlossenem Deckel und mit einem ersten Taumelantrieb und einer ersten Taumelvorrichtung,

- Fig. 34: einen Schnitt durch das Taumelpumpenbasismodul mit einem in der Aufnahme aufgenommenen Pumpenmodul bei geschlossenem Deckel und mit einem zweiten Taumelantrieb und einer zweiten Taumelvorrichtung,

- Fig. 35: einen Schnitt durch das Taumelpumpenbasismodul mit einem in der Aufnahme aufgenommenen Pumpenmodul bei geschlossenem Deckel und mit einem dritten Taumelantrieb und einer dritten Taumelvorrichtung,

- Fig. 36: einen Schnitt durch das Taumelpumpenbasismodul mit einem in der Aufnahme aufgenommenen Pumpenmodul bei geschlossenem Deckel und mit einem vierten Taumelantrieb und einer vierten Taumelvorrichtung, und

- Fig. 37: einen Schnitt durch das Taumelpumpenbasismodul mit einem in der Aufnahme aufgenommenen Pumpenmodul bei geschlossenem Deckel und mit einem vierten Taumelantrieb und einer vierten Taumelvorrichtung.

### Beschreibung der Ausführungsarten

Gleiche oder einander entsprechende Bauteile sind in den Figuren mit denselben Bezugszeichen versehen.

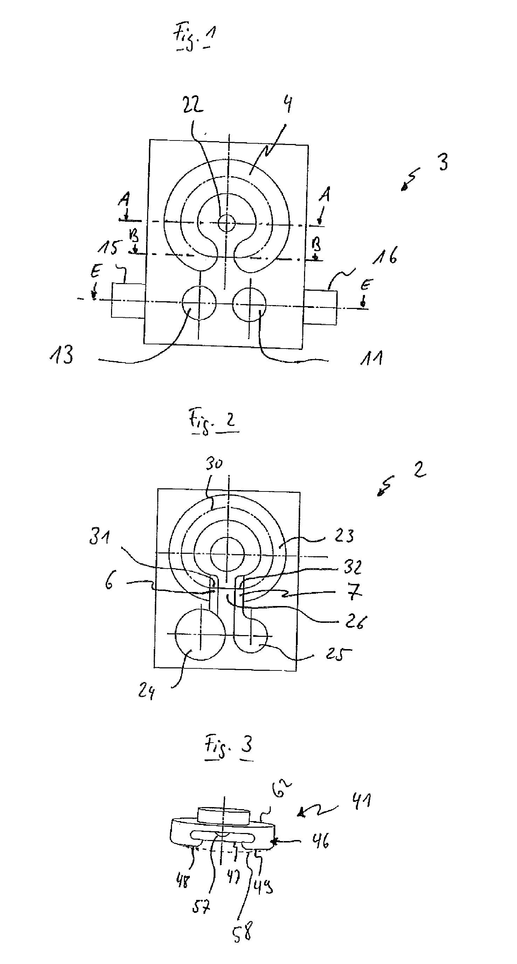

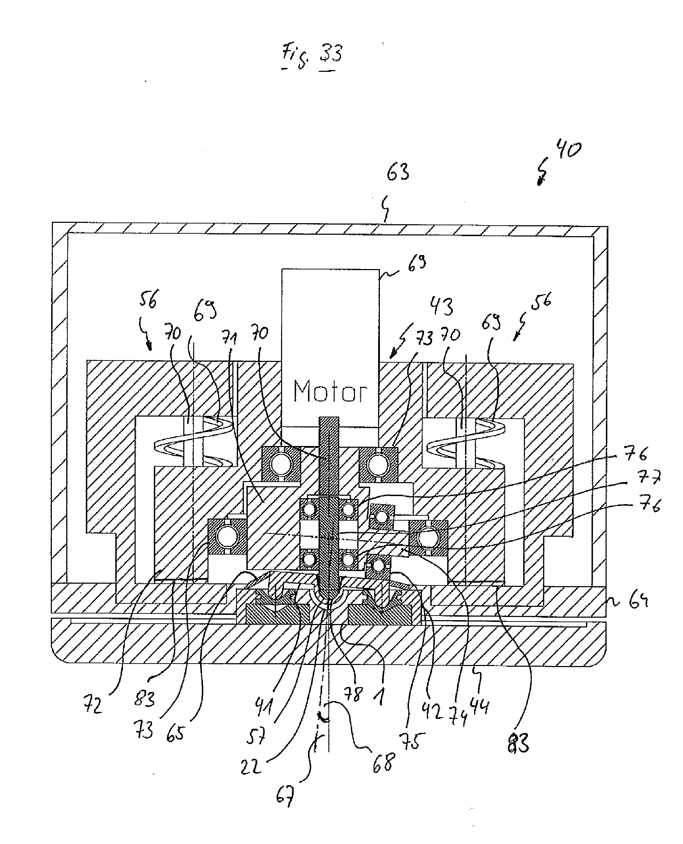

Die Figuren 1, 2, 4 und 5 zeigen eine erste Ausführungsform eines Pumpmoduls 1 bzw. einzelne Elemente des Pumpmoduls 1. Das Pumpmodul 1 ist für die Verwendung mit einem Taumelpumpenbasismodul ausgebildet, wie beispielsweise in Fig. 33 dargestellt. Eine Verwendung des Pumpmodul 1 mit anderen Pumpentypen soll dadurch nicht ausgeschlossen sein, obwohl die genannte Verwendung bevorzugt ist. Pumpmodul und Taumelpumpenbasismodul bilden ein Taumelpumpensystem, mittels dem ein Fluid, also ein Gas oder eine Flüssigkeit, förderbar ist.

Das Pumpmodul umfasst ein Basisteil 2, ein Deckelteil 3 und eine elastisch verformbare Membran 4. Basisteil 2 und Membran 4 bilden einen linienförmigen, zumindest abschnittsweise gekrümmten Pumpkanal 5. Das Basisteil 2 weist einen Pumpkanaleingang 6 und einen Pumpkanaleingang 7 auf. Der Pumpkanaleingang 6 und der Pumpkanalausgang 7 sind mit dem Pumpkanal 5 zum Zuführen und Abführen eines Fluids in den bzw. aus dem Pumpkanal 5 verbunden. Durch die taumelnde Verformung der Membran 4 ist durch die damit bewirkte umlaufende lokale Kompression des Pumpkanals 5 ein Fluid durch den Pumpkanal 5 vom Pumpkanaleingang 6 zum Pumpkanalausgang 7 pumpbar.

Durch Umkehrung der Taumelbewegung ist es grundsätzlich ebenfalls möglich, ein Fluid vom Pumpkanalausgang 7 zum Pumpkanaleingang 6 zu pumpen.

In diesem Ausführungsbeispiel umfasst das Basisteil 2 eine Rinne 23. Die Rinne 23 bildet mit der im entspannten Zustand ebenen Unterseite der in diesem Ausführungsbeispiel flachen und beidseitig ebenen Membran 4 den Pumpkanal 5 aus. Die Rinne 23 hat einen leicht gewölbtes Profil derart, dass die Membran 4 durch eine Taumelvorrichtung, beispielsweise durch eine in Fig. 3 abgebildete Taumelscheibe 41, abdichtend an die Oberfläche der Rinne 23 gepresst werden kann, ohne dass die Membran 4 übermäßigen Scherkräften ausgesetzt ist. Alternativ ist es ebenfalls möglich, die Membran 4 mit einer Rinne auszubilden und das Basisteil 2 mit einer ebenen Oberfläche zu gestalten, oder sowohl Membran 4 als auch Basisteil 2 jeweils mit einer von einer ebenen Fläche abweichenden Form für die Bildung des Pumpkanals 5 auszubilden.

Der Pumpkanal 5 ist nicht vollständig umlaufend ausgebildet, sondern durch einen Steg 26 unterbrochen. Der Steg 26 bewirkt, dass es nur eine Möglichkeit für das Fluid gibt, zwischen Pumpkanaleingang 6 und Pumpkanalausgang 7 zu strömen.

Pumpkanaleingang 6 und Pumpkanalausgang 7 grenzen unmittelbar an jeweils eine Seite des Stegs 26, wodurch das Volumen des Pumpkanals 5 für die Förderung des Fluids nahezu vollständig ausgenutzt werden kann.

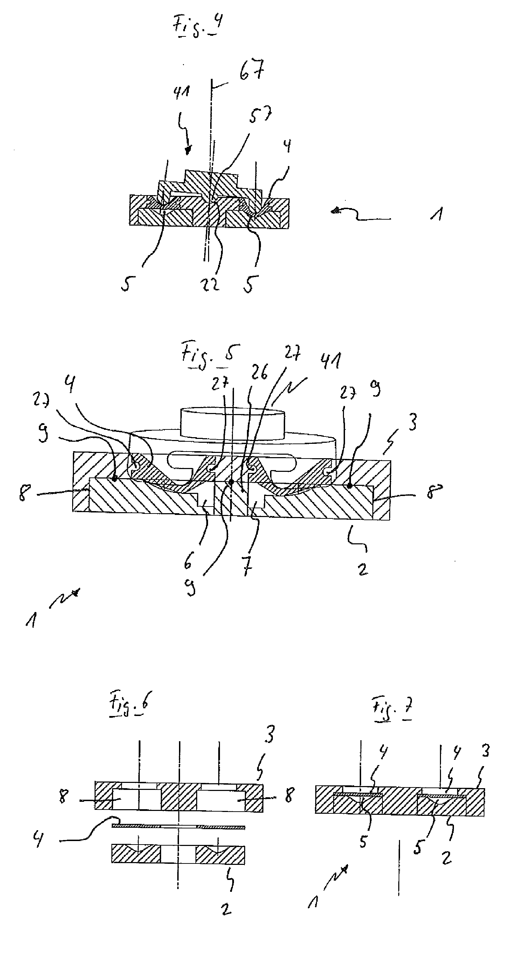

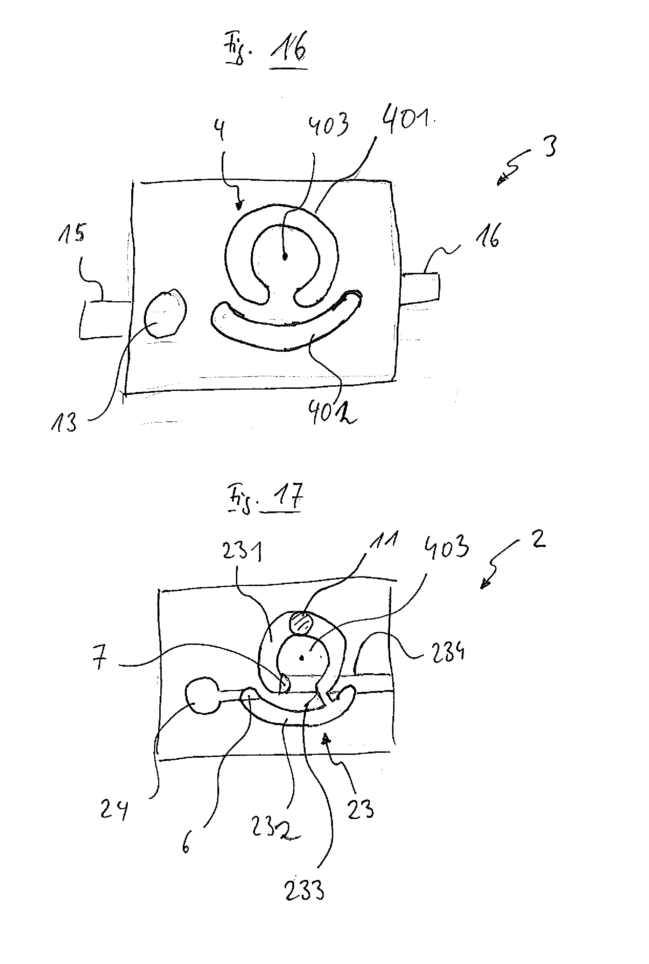

Der Pumpkanaleingang 6 und der Pumpkanalausgang 7 sind hier als nutförmige, Aussparungen ausgebildet, siehe Fig.5, die am jeweiligen Ende des Pumpkanals 5 an dessen Boden angeordnet sind. Alternativ wären auch anderen Formen möglich, beispielsweise Kanäle mit runden oder elliptischen Öffnungen. Die nutförmigen Aussparungen gehen senkrecht zum Pumpkanal 5 ab und enden in einer ersten zylinderförmigen Aussparung 24 bzw. einer zweiten zylinderförmigen Aussparungen 25. Die erste zylinderförmige Aussparung ist Teil einer Ventilkammer 12, die zweite zylinderförmige Aussparung ist Teil einer Druckmesskammer 10, siehe beispielsweise Fig. 16.

Die Membran 4 ist mit dem Deckelteil 3 verbunden. Verschiedene Arten von Verbindungen sind möglich, beispielsweise stoffschlüssig, insbesondere durch Kleben oder Angiessen, wobei letzteres auch Anspritzen umfassen soll, oder kraftschlüssig durch Klemmen. Bevorzugt ist, dass die Membran 4 an dem Deckelteil 3 angespritzt ist, wie es in diesem Ausführungsbeispiel umgesetzt ist. Zur besseren Haftung der Membran 4 am Deckelteil 3 umfasst das Deckelteil 3 einen an der zum Pumpkanal 5 hin gerichteten Innenseite entlanglaufenden Befestigungsvorsprung 27, siehe Fig. 5, der von der Membran 4 ober- und unterseitig umgriffen ist.

Eine alternative Ausführungsform eines Pumpmoduls 1, bei dem die Membran 4 durch Klemmen befestigt ist, zeigen die Figuren 6 und 7. In dieser Variante ist die Membran 4 ein separates flaches Element mit ebener Unter- und Oberseite, das zwischen Basisteil 2 und Deckelteil 3 eingeklemmt ist.

Alternativ kann die Membran 4 auch mit dem Basisteil 2 verbunden sein, insbesondere mit den im Zusammenhang mit dem Deckelteil 3 genannten Verbindungsarten.

Basisteil 2 und Deckelteil 3 sind hinterschnittsfrei ausgebildet. Basisteil 2 und Deckelteil 3 sind aufgrund dessen ohne aufwendiges Werkzeug kostengünstig herstellbar. Insbesondere kann eine aufwendige Entformung entfallen.

Das Deckelteil 3 umfasst eine Aussparung 8, in die das Basisteil 2 unter Bildung eines Formschlusses eingesetzt ist, siehe Fig. 5. Der Formschluss gewährleistet, dass die relative Position von Deckelteil 3 und Basisteil 2 definiert ist. Beim Zusammenbau von Deckelteil 3 und Basisteil 2 lassen sich Herstellungstoleranzen minimieren, so dass eine Vielzahl von Pumpmodulen mit im Wesentlichen gleichen Eigenschaften herstellbar ist. Alternativ ist es ebenfalls möglich, dass das Basisteil 2 eine Aussparung aufweist, in die das Deckelteil 3 unter Ausbildung eines Formschlusses eingesetzt ist oder eingreift, oder sowohl Deckelteil 3 als auch Basisteil 2 jeweils zumindest eine Aussparung aufweist, in die das andere Element formschlüssig eingreift. In diesem Ausführungsbeispiel umfasst das Deckelteil 3 auf seiner Rückseite eine quaderförmige Aussparung, in die das Basisteil 2, das eine zur Aussparung korrespondierende quaderförmige Außenform aufweist, eingesetzt ist.

Basisteil 2 und Deckelteil 3 sind aus einem harten Material, vorzugsweise einem Kunststoff, insbesondere einem Thermoplast, hergestellt. Als Material eignet sich beispielsweise POM (Polyoxymethylen), PC (Polycarbonat) oder COC (Cycloolefin Copolymer). Basisteil 2 und Deckelteil 3 sind hier als einteilige, insbesondere einstückige Spritzgussteile hergestellt. Basisteil 2 und Deckelteil 3 bestehen aus demselben Material, wodurch eine stoffschlüssige Verbindung zwischen Basisteil 2 und Deckelteil 3 einfach und kostengünstig herstellbar ist, vorzugsweise mittels eines Schweißprozesses, beispielsweise eines Ultraschallschweißprozesses oder Laserschweißprozesses. Im Falle von Laserschweißen ist bevorzugt, dass entweder Basisteil 2 oder Deckelteil 3 transparent ist, während das andere Teil für den Laserstrahl zumindest im Schweißbereich absorbierend ist. Basisteil 2 und Deckelteil 3 können aber auch durch andere Verbindungstechniken, beispielsweise durch Kleben oder Klemmen, miteinander verbunden werden.

Die Membran 4 ist vorzugsweise einteilig (siehe zweite Ausführungsform, Figuren 6, 7) oder im Deckelteil 3 integriert (wie beispielsweise bei der hier beschriebenen ersten Ausführungsform). Vorzugsweise ist die Membran 4 einstückig. Die Membran 4 ist vorzugsweise ein Elastomer, vorzugsweise ein thermoplastisches Elastomer, beispielsweise ein EPDM (Ethlyen-Propylen-Dien-Kautschuk), Ethlyen-Propylen-Kautschuk oder Silikonkautschuk. Membran 4 und Decketeil 3 der ersten Ausführungsform des Pumpenmoduls 1 sind mittels eines 2K-Spritzgussprozesses (2-Komponenten-Spritzguss) hergestellt.

Die für Basisteil 2, Deckelteil 3 und Membran 4 verwendeten Werkstoffe sind kostengünstig und lassen sich präzise verarbeiten. Aufgrund dessen kann das Pumpenmodul 1 kostengünstig, mit reproduzierbaren Abmessungen und mit einer hohen Robustheit hergestellt werden. Des Weiteren können kostengünstig und platzsparend mehrere Funktionalitäten in das Pumpmodul 1 integriert werden, wie später noch beschrieben wird.

Das Pumpenmodul umfasst des Weiteren eine Dichtung 9, siehe Fig. 5. Die Dichtung 9 ist zwischen Basisteil 2 und Deckelteil 3 angeordnet. Die Dichtung 9 dichtet die Fluid-führenden Bereiche des Pumpmoduls 1 gegen den Außenraum ab. Die Dichtung 9 umgibt umlaufend sowohl den Pumpkanal 5 als auch die Druckmesskammer 10 und die Ventilkammer 12, siehe auch Fig. 16. Die Dichtung 9 ist hier durch eine Dichtlippe gebildet. Die Dichtlippe kann an das Deckelteil 3 oder an das Basisteil 2 angegossen oder lose angeordnet sein. Bevorzugt ist, dass die Dichtlippe im Rahmen eines 2K-Spritzgusses angespritzt wird, beispielsweise im Rahmen des Herstellungsprozesses der Membran 4 an das Deckelteil 3 mit angespritzt wird. Die Dichtlippe besteht im letzteren Fall vorzugsweise aus demselben Material wie die Membran 4. Alternativ kann die Dichtlippe oder ein Teil der Dichtlippe an das Basisteils 2 angespritzt werden.

Alternativ können anstatt einer Dichtlippe beispielsweise auch ein oder mehrere Dichtringe oder andere Dichtungsmittel verwendet werden.

Im Falle dessen, dass Basisteil 2 und Deckelteil 3 mittels Schweißens, insbesondere mittels Laserschweißens, verbunden werden, können die Dichtungen auch im Rahmen des Schweißprozesses erzeugt werden. Dabei werden die fluidführenden Bereiche so umfahren, dass sie untereinander und gegen den Außenraum durch die Schweißlinie gedichtet werden.

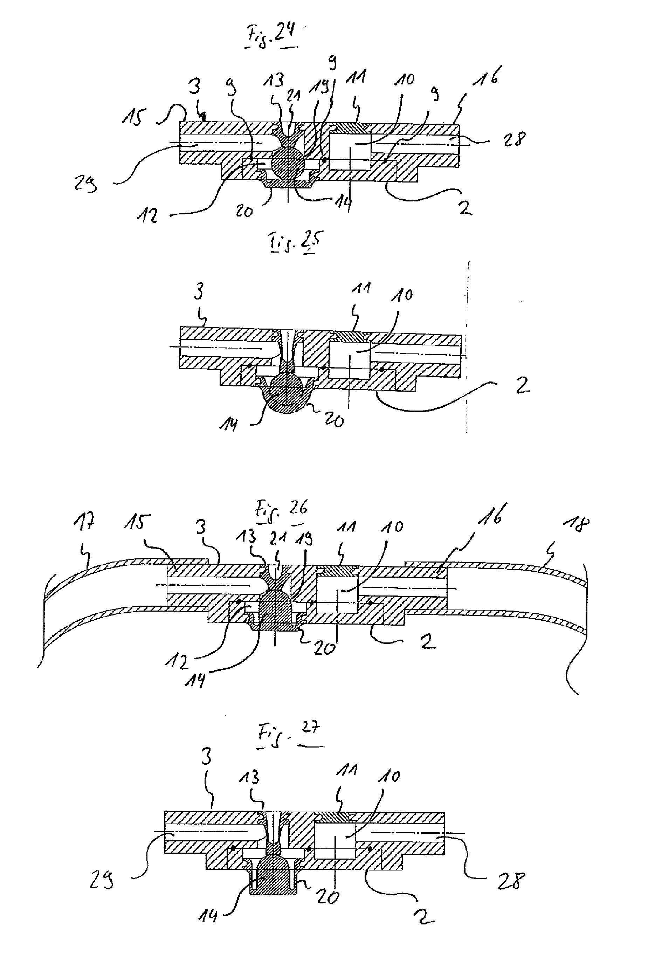

Das Deckelteil 3 und das Basisteil 2 bilden eine Druckmesskammer 10 aus, siehe Fig. 24-27. Die zylinderförmige Aussparung 25 des Basisteils 2, siehe Fig. 2, geht in eine zylinderförmige Aussparung mit entsprechendem Durchmesser des Deckelteils 3 über. Die zylinderförmige Aussparung des Deckelteils 3 ist mit einem ebenfalls durch das Deckelteil 3 ausgebildeten Auslasskanal 28 verbunden, über den das Fluid das Pumpmodul 1 verlassen kann.

Die Druckmesskammer 10 umfasst einen flexiblen Wandabschnitt 11, der sich durch einen in der Druckmesskammer 10 ausgeübten Druck eines Fluids verformen kann. Im Betrieb des Pumpmoduls 1 erzeugt jeder Pumpzyklus mit einem Fluid eine Verformung des Wandabschnitts 11. Über den Grad der Verformung des Wandabschnittes 11 lassen sich Rückschlüsse auf den Druck in der Druckmesskammer 10 und damit auf den Druck des Fluids ziehen. Insbesondere ein Überdruck, der beispielsweise im Falle einer stromabwärts liegenden Okklusion auftritt, oder ein Druckverlust bei einer Beschädigung einer mit dem Pumpmodul 1 verbundenen Leitung oder Schlauches lassen sich auf diese Weise erkennen. Des Weiteren besteht die Möglichkeit, dass im Falle dessen, dass das Pumpmodul 1 mit einem Fluid-enthaltenden Beutel verbunden ist (was eine übliche Anwendung ist), über eine Druckmessung erkannt werden kann, ob der Beutel leer ist. Die Druckmesskammer 10 kann außerhalb des Pumpkanals 5 angeordnet sein, wie in diesem Ausführungsbeispiel gezeigt, oder auch zwischen Pumpkanaleingang 6 und Pumpkanalausgang 7 angeordnet und entweder Teil des Pumpkanals 5 sein oder mit diesem, beispielsweise über einen Kanal, in Verbindung stehen. Letztere Varianten ermöglichen die Messung von Drücken sowohl stromaufwärts als auch stromabwärts, was für die Erkennung einer Okklusion, eines Schlauchfehlers oder eines leeren Beutels benutzt werden kann.

Auch kann der Druck innerhalb des Pumpkanals 5 oder eines Abschnitts der Pumpkanals 5 gemessen werden, wenn Pumpkanaleingang 6 und -Ausgang 7 gleichzeitig geschlossen sind. Dies ermöglicht es, insbesondere im Falle eines kleinen Volumens des Pumpkanals 5, beispielsweise über ein Vergleich der gemessen Drücke mit Referenzwerten, auf das Fluidvolumen im Pumpkanal 5 und das Vorhandensein von Luftblasen im Fluid zu erkennen, insbesondere Luftblasen in der Größenordnung der Pumpkammer oder größer. Auf diese Weise können etablierte Messverfahren zur Luftdetektion wie zum Beispiel mittels der Messung der Dämpfung einer das Fluid durchlaufenden Ultraschallwelle kostengünstig ersetzt werden.

Alternativ oder zusätzlich können auch zwei Druckmesskammern 10 vor und nach dem Pumpkanal 5 angeordnet werden, um stromaufwärts und stromabwärts Drücke messen zu können. Damit lässt sich zum einen beispielsweise ein leerer Flüssigkeitsbeutel erkennen. Des Weiteren lassen sich auch einzelne Luftblasen in der Flüssigkeit detektieren oder stromab-oder stromaufwärts liegenden Okklusionen erkennen.

Der flexible Wandabschnitt 11 ist in diesem Ausführungsbeispiel als Membran ausgebildet, was erfindungsgemäß bevorzugt ist. Das Deckelteil 3 weist oberhalb der Druckmesskammer 10 eine kreisförmige Aussparung auf, in die der flexible Wandabschnitt 11 angeordnet ist. Vorzugsweise ist der flexible Wandabschnitt aus demselben Material, das für die Membran 4 bevorzugt ist. Aus Gründen einer kostengünstigen Herstellung ist es besonders vorteilhaft, flexiblen Wandabschnitt 11 und Membran 4 im Rahmen eines gemeinsamen Prozesses zu erzeugen, insbesondere im Rahmen eines 2K-Spritzgussprozesses in Verbindung mit der Herstellung des Deckelteils 3. Das Deckelteil 3 weist an seiner Innenseite der Aussparung für den flexiblen Wandabschnitt 11 einen Vorsprung auf, der durch den flexiblen Wandabschnitt ober- und unterseitig umgriffen wird, wodurch die Verbindung von Wandabschnitt 11 und Deckelteil 3 erhöht wird.

Alternativ oder zusätzlich wäre es möglich, den flexiblen Wandabschnitt 11 im Basisteil 2 vorzusehen. Die Aussparung für den flexiblen Wandabschnitt 11 im Deckelteil 3 wäre entsprechend am Boden des Basisteils 2 vorgesehen.

Der flexible Wandabschnitt 11 ist von Außen direkt zugänglich. Eine Verformung des Wandabschnitts 11 kann damit ohne Behinderung durch andere Teile des Pumpmoduls 1 ermittelt werden. In diesem Ausführungsbeispiel bildet der unverformte flexible Wandabschnitt 11 mit der Oberfläche des Deckelteils 3 eine ebene Fläche aus.

Alternativ oder zusätzlich kann eine Druckmessung des Fluids über die Membran 4 erfolgen. Hierdurch lässt sich der Druck in dem Pumpkanal 5 bestimmen.

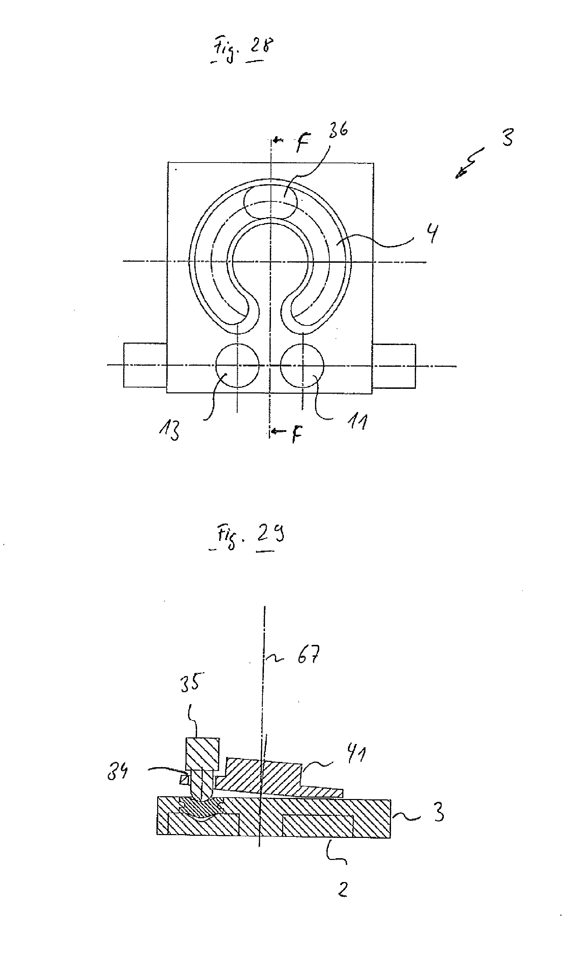

Eine Ausführungsform, die eine Vorrichtung zur Messung des Drucks innerhalb des Pumpkanals 5 über die Membran 4 darstellt, zeigen die Figuren 28 und 29.

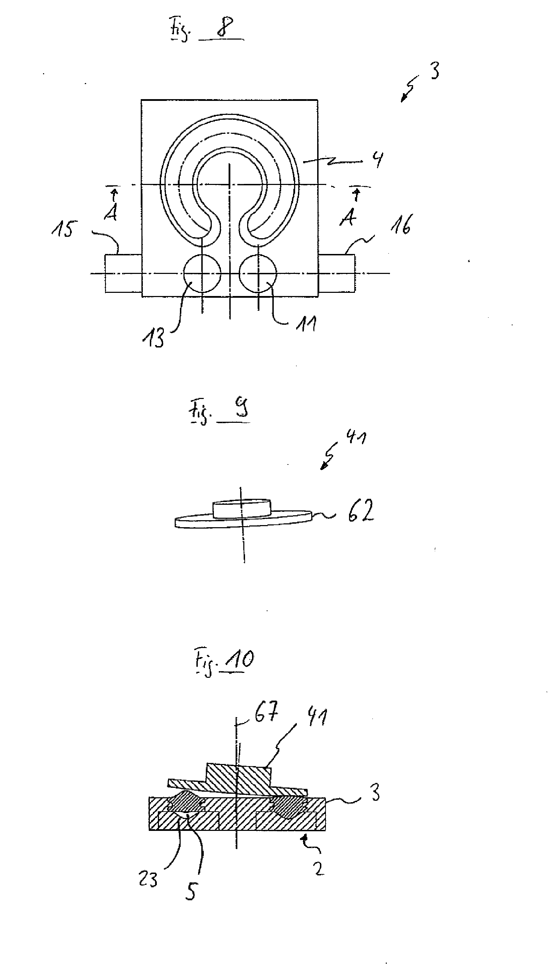

Die Taumelvorrichtung 41 weist in einem Abschnitt gegenüberliegend der Membran 4 eine Aussparung 34 auf, durch die ein Druckmessmittel 35 durchgreift, hier ein beweglicher, federnd vorgespannter starrer Messfinger, der mit einem Drucksensor (nicht dargestellt) verbunden ist. Die Verformung der Membran 4 wird über das Druckmessmittel 35 erfasst und zur Ermittelung des Druckes innerhalb des Pumpkanals 5 verwendet. Verwendet man eine Membran 4 mit einem Wulst (siehe beispielsweise Figuren 8 und 10), so ist der Wulst vorzugsweise in einem Messbereich 36, siehe Fig. 28, in dem das Druckmessmittel die Membran 4 kontaktiert, unterbrochen, um die Empfindlichkeit der Druckmessung zu erhöhen. Neben der Änderung der Wandstärke und/oder der Form kann zusätzlich oder alternativ die Membran 4 in diesem Bereich auch aus einem anderen Material ausgebildet sein, beispielsweise aus einem Material mit erhöhter Elastizität.

Das Druckmessmittel 35 ist hier von der Taumelbewegung der Taumelvorrichtung 41 entkoppelt. Es ist fest im Gehäuse des Taumelpumpenbasismoduls 40 montiert, um die Amplitude des Messfingers relativ zur Membran 4 zu minimieren. Alternativ ist es ebenfalls möglich, das Druckmessmittel 35 zumindest teilweise in der Taumelvorrichtung 41 zu integrieren.

In Abhängigkeit der periodisch umlaufenden Quetschung der Membran 4 kann auf diese Weise der Druck stromabwärts, stromaufwärts und bei geschlossenem Eingang 6 und Ausgang 7 auch innerhalb des Pumpkanals 5 gemessen werden.

Das Pumpenmodul 1 umfasst in der gezeigten Ausführungsform neben der Druckmesskammer 10 eine Ventilkammer 12. Die Ventilkammer 12 ist mit dem Pumpkanal 5 verbunden, so dass durch Pumpkanal 5 und Ventilkammer 12 ein Fluid förderbar ist. Die Verbindung zwischen Pumpkanal 5 und Ventilkammer 12 ist über den Pumpkanaleingang 6 hergestellt, siehe Fig. 2 und Fig. 24. Das Pumpmodul 1 umfasst einen beweglichen Ventilkörper 14, der in der Ventilkammer 12 angeordnet ist. Der Ventilkörper kann eine Ruhestellung einnehmen, in der der Ventilkörper 14 die Ventilkammer 12 gegen einen Durchtritt des Fluids verschließt, siehe Fig. 24 oder Fig. 26, oder eine Betätigungsstellung einnehmen, in der der Ventilkörper 14 einen Durchfluss durch die Ventilkammer 12 freigibt, siehe Fig. 25 oder Fig. 27. Ein erster Wandabschnitt 13 der Ventilkammer 12 ist flexibel ausgebildet und steht in Wirkverbindung mit dem Ventilkörper 14, so dass durch Verformung des ersten Wandabschnitts 13 der Ventilkörper in die Betätigungsstellung bringbar ist, um den Durchfluss des Fluids durch die Ventilkammer 12 zu ermöglichen.

Der Ventilkörper ist in der Ruhestellung gegen einen Anschlag 19 der Ventilkammer federnd vorgespannt. Dadurch ist ein gewisser Mindestdruck notwendig, damit der Ventilkörper 14 die Ruhestellung verlässt und das Ventil sich öffnet. Die Vorspannung ist derart eingestellt, dass das Fluid einen Mindestdruck von ≥ 2 bar, vorzugsweise einen Mindestdruck von ≥ bar aufweisen muss, um den Ventilkörper 14 vom Anschlag 19 wegzudrücken. Das Pumpmodul 1 ist somit in seiner Grundstellung geschlossen. Erst durch eine Betätigung des Ventilkörpers 14 ist ein Fluidtransport durch das Pumpmodul 1 möglich. Das Ventil gewährleistet damit, dass ein Fluid nicht ungewünscht das Pumpmodul 1 passiert, beispielsweise im Falle eines an das Pumpmodul 1 angeschlossenen Beutels Flüssigkeit unerwünscht den Beutel verlässt. Ein derartiges Ventil wird auch als Anti-Free-Flow-Ventil bezeichnet. Des Weiteren bewirkt die federnde Vorspannung des Ventilkörpers 14, dass der Ventilkörper 14 in die Ruhestellung gepresst wird, wodurch bei nachlassendem Druck des Fluids oder des ersten flexiblen Wandabschnitts 13 auf den Ventilkörper 14 der Ventilkörper 14 in seine Ruhestellung zurückkehrt.

Wird mittels des Pumpmoduls 1 ein Fluid in umgekehrter Richtung gefördert bzw. das Pumpmodul 1 in anderer Durchflussrichtung betrieben, was ausdrücklich ebenfalls eine mögliche Verwendung des Pumpmoduls 1 darstellt, wird nicht nur durch die Vorspannung des Ventilkörpers 14 sondern auch durch das Fluid selbst der Ventilkörper 14 gegen den Anschlag 19 und damit in seine Ruhestellung gedrückt. Dadurch bleibt das Ventil ohne ein aktives Öffnen unabhängig vom Druck des Fluids geschlossen.

Die Ventilkammer 12 umfasst einen zweiten Wandabschnitt 20, wobei der zweite Wandabschnitt 20 elastisch verformbar ist. Der zweite Wandabschnitt 20 ist in Wirkverbindung mit dem Ventilkörper 14 und spannt den Ventilkörper 14 federnd gegen den Anschlag 19 vor. In einer ersten Variante, die in den Figuren 24 und 25 dargestellt ist, ist der zweite Wandabschnitt 20 und der Ventilkörper 14 zweiteilig ausgebildet. In einer zweiten Variante, die in den Figuren 26 und 27 dargestellt ist, ist der zweite Wandabschnitt 20 und der Ventilkörper 14 einteilig, in diesem Falle sogar einstückig ausgebildet.

Der erste Wandabschnitt 13, der zweite Wandabschnitt 20 und der Ventilkörper 14 sind vorzugsweise aus halbstarren ("semi-rigid") oder weichen elastisch verformbaren Materialen ausgebildet. Bevorzugte Materialien sind thermoplastische Kunststoffe, thermoplastische Elastomere oder silikonhaltige Materialien. Insbesondere können die gleichen Materialien verwendet werden, die für die Herstellung der Membran 4, des flexiblen Wandabschnitts 11 der Druckmesskammer 10 oder der Dichtlippe 9 verwendet werden. Die Anzahl unterschiedlicher Materialien und die Anzahl der Prozessschritte zur Herstellung des Pumpmoduls 1 lassen sich hierdurch verringern, wodurch das Pumpmodul 1 kostengünstiger herstellbar ist. Insbesondere können der erste Wandabschnitt 13 und der zweite Wandabschnitt 20 im Rahmen eines 2K-Prozesses mit einem weiteren Teil des Pumpmoduls 1, insbesondere mit dem Basisteil 1 oder mit dem Deckelteil 3 hergestellt werden.

In diesem Ausführungsbeispiel wird die Ventilkammer 12 durch eine zylinderförmige Aussparung 24 des Basisteils 2, siehe Fig. 2, gebildet, die in eine zylinderförmige Aussparung mit verringertem Durchmesser des Deckelteils 3 übergeht. Die zylinderförmige Aussparung des Deckelteils 3 ist mit einem ebenfalls durch das Deckelteil 3 ausgebildeten Einlasskanal 29 verbunden, über den das Fluid in das Pumpmodul 1 eintreten kann, siehe Fig. 24.

Das Decketeil 3 weist oberhalb der Ventilkammer 12 eine runde Aussparung auf, in der der erste flexible Wandabschnitt 13 angeordnet ist. An dem Innenrand der Aussparung umfasst das Deckelteil 3 einen Vorsprung, der durch den flexiblen Wandabschnitt 13 beidseitig umgriffen ist, wodurch die Haltbarkeit der Verbindung des ersten Wandabschnitts 13 mit dem Deckelteil 3 verbessert ist.

Das Basisteil 2 weist unterhalb der Ventilkammer 12 eine runde Aussparung auf, in der der zweite flexible Wandabschnitt 20 angeordnet ist. An dem Innenrand der Aussparung umfasst das Basisteil 2 einen Vorsprung, der durch den zweiten Wandabschnitt 20 beidseitig umgriffen ist, wodurch die Haltbarkeit der Verbindung des zweiten Wandabschnitts 20 mit dem Basisteil 2 verbessert ist.

Der Anschlag 19 wird durch eine Stufe gebildet, die dadurch entsteht, dass der zylinderförmige Abschnitt der Ventilkammer 12 im Basisteils 2 einen größeren Durchmesser hat als der zylinderförmige Abschnitt der Ventilkammer 12 im Deckelteil 3.

Der Ventilkörper 14 ist im Bereich der Ventilkammer 12 angeordnet, der sich im Basisteil 12 befindet. Der zweite flexible, elastische Wandabschnitt 20 ist in der Ruhestellung des Ventilkörpers 14 bereits verformt, wodurch der Anpressdruck erzeugt wird, der den Ventilkörper 14 dichtend gegen den Anschlag 19 presst. Durch Verformung des ersten Wandabschnitts 13, vorzugsweise in einer Verformungsrichtung, die in Richtung des Ventilkörpers 14 gerichtet ist, in diesem Falle senkrecht zur Ebene des ersten Wandabschnitts 13 und des Deckelteils 3, lässt sich der Ventilkörper 14 in die Betätigungsstellung überführen, siehe Fig. 25 und Fig. 26. Der zweite flexible Wandabschnitt wölbt sich mit Übergang des Ventilkörpers 14 in die Betätigungsstellung mehr und mehr nach Außen. Nach Reduzierung des Drucks auf den ersten Wandabschnitt 13 führen die Rückstellkräfte des zweiten Wandabschnitts 20 zu einer Rückführung des Ventilkörpers 14 in seine Ruhestellung.

Der erste Wandabschnitt 13 ist in diesem Ausführungsbeispiel als Teil der Außenwand des Pumpmoduls 1 ausgebildet, so dass der erste Wandabschnitt 13 von Außen ohne Behinderung betätigbar ist. Auch der zweite Wandabschnitt 20 ist als Teil der Außenwand des Pumpmoduls 1 ausgebildet. Dies ermöglicht es, ein Pumpmodul mit einer kompakten Größe herzustellen.

Der erste Wandabschnitt 13 weist eine von Außen zugängliche Vertiefung 21 auf für den Eingriff eines Vorsprungs 51, beispielsweise eines Stiftes oder eines Steges. Auf diese Weise lässt sich ein Missbrauch des Ventils verhindern, insbesondere ein unbeabsichtigtes Öffnen des Ventils. Des Weiteren kann eine derartige Vertiefung als Positionierhilfe dienen, um das Pumpmodul in einer richtigen Position mit einem Taumelpumpenbasismodul zu verbinden. Alternativ kann der erste Wandabschnitt 13 auch in anderen Formen, beispielsweise eben oder als Erhöhung über der Oberfläche des Deckelteils 3 hervorstehend ausgebildet werden. Letztere Variante ermöglicht eine einfache Betätigung des Ventils auch per Hand, was vorteilhaft ist, wenn Bedarf besteht, das Ventil auch dann zu öffnen, wenn das Pumpenmodul noch nicht mit dem Taumelpumpenbasismodul verbunden ist.

Alternativ können die Funktionen von Basisteil 2 und Deckelteil 3 vertauscht sein, also der zweite Wandabschnitt 20 und der Ventilkörper 14 im Deckelteil 3 und der erste Wandabschnitt 13 im Basisteil 2 angeordnet sein. Auch der Einlasskanal 29 und der Auslasskanal 28 lassen sich teilweise oder vollständig durch das Basisteil 2 ausbilden. Andere Formen der Ventilkammer 12 und/oder der Druckmesskammer 10 sind grundsätzlich ebenfalls möglich, beispielsweise elliptische oder quaderförmige Formen.

Die beschriebene Ventilkammer 12 eignet sich nicht nur in Kombination mit einem Pumpkanal 5, der mit Hilfe einer Membran 4 gebildet ist, sondern grundsätzlich auch für andere Pumpaufbauten. Beispielsweise könnte als Pumpkanal 5 ein elastisch verformbarer Schlauch verwendet werden. Des Weiteren kann der erfindungsgemäße Aufbau der Ventilkammer 12 unabhängig von dem Pumpprinzip verwendet werden. So kann beispielsweise der erfindungsgemäße Aufbau der Ventilkammer 12 Teil einer linearen peristaltischen Fingerpumpe, einer Rollenpumpe oder einer Membranpumpe sein. Selbiges gilt für die Druckmesskammer 10, insbesondere in Kombination mit der Ventilkammer 12. Besonderer Vorteil der Kombination eines Pumpprinzips, das eine Membran verwendet, mit der Ventilkammer 12 und auch der Druckmesskammer 10 ist, dass einzelne Elemente des Pumpmoduls mit mehrere Funktionen ausgestattet werden können, so dass das Pumpmodul 1 kostengünstig, in einer kompakten Form und mit einer hohen mechanischen Präzision herstellbar ist.

Das Deckelteil 3 umfasst einen ersten Anschluss 15 und einen zweiten Anschluss 16 zum Anschließen eines Schlauches. Der erste Anschluss 15 ist mit dem Pumpkanaleingang 6 verbunden, der zweite Anschluss 16 mit dem Pumpkanalausgang 7. In diesem Ausführungsbeispiel sind der erste Anschluss 15 und der zweite Anschluss 16 als rohrförmige Stutzen ausgebildet, über die jeweils ein Schlauchabschnitt 17, 18 übergezogen, siehe Fig. 26, und optional mit dem Stutzen zwecks besserer Festigkeit der Verbindung stoffschlüssig verbunden werden können, beispielsweise durch Kleben oder Verschweißen. Ein Abschnitt des Einlasskanals 29 ist mit dem ersten Anschluss 15 ausgebildet, ein Abschnitt des Auslasskanals 28 mit dem zweiten Anschluss 16. Ein Fluid, das über den Einlasskanal 29 in das Pumpmodul 1 eingeleitet wird, durchströmt somit nach dem Einlasskanal 29 die Ventilkammer 12, den Pumpkanaleingang 6, die Pumpkammer 5, den Pumpkanalausgang 7 und die Druckmesskammer 10, bevor es über den Auslasskanal 28 aus dem Pumpmodul 1 austritt.

Ebenfalls ist es möglich, dass ein Fluid das Pumpmodul 1 in anderer Richtung durchströmt. In diesem Falle bildet das Ventil des Pumpmoduls 1 ein Sperrventil, das unabhängig von dem Druck des Fluids den Durchfluss des Fluids zum Einlasskanal 29 sperrt und allein durch Betätigung des Ventilkörpers 14 über den ersten flexiblen Wandabschnitt 13 den Durchfluss freigibt.

Der erste Anschluss 15 und/oder der zweite Anschluss 16 kann alternativ in anderen Formen, beispielsweise als Hülse, in denen ein Schlauchabschnitt einsteckbar ist, ausgebildet werden.

Der Pumpkanal 5 kann rein kreisbogenförmig ausgebildet sein, oder ist, wie in den Ausführungsbeispielen gezeigten Pumpmodulen 1, abweichend von einer reinen Kreisbogenform ausgebildet. Auf diese Weise lässt sich der unerwünschte Zustand, dass während des Betriebs des Pumpenmoduls 1 zwischen Pumpkanaleingang 6 und Pumpkanalausgang 7 eine kurzfristige offene Verbindung besteht (ein "Kurzschluss"), in der das Fluid ungehindert oder nicht ausreichend gehindert durch den Pumpkanal 5 strömen kann, verhindern.

Die beispielsweise in den Figuren 1 und 2 gezeigte erste Ausführungsform des Pumpmoduls 1 umfasst einen Pumpkanal 5, der einen kreisförmigen Abschnitt 30, einen ersten geraden Abschnitt 31 und einen zweiten geraden Abschnitt 32 aufweist. Der kreisförmige Abschnitt ist zwischen dem ersten geraden Abschnitt und dem zweiten geraden Abschnitt angeordnet. Der Pumpkanaleingang 6 liegt im ersten geraden Abschnitt 31, der Pumpkanalausgang 7 im zweiten geraden Abschnitt 32 des Pumpkanals 5. Dadurch, dass der erste Abschnitt 31 und der zweite Abschnitt geradlinig ausgebildet sind, ist eine Ausführungsform verwirklicht, in der durch eine Taumelvorrichtung 41, beispielsweise durch eine Taumelscheibe wie in Fig. 3 gezeigt, der Pumpkanal 5 am Pumpkanaleingang 6 und am Pumpkanal 7 nahezu gleichzeitig dichtend abgequetscht werden kann, siehe Fig. 5. Der erste Abschnitt 31 und der zweite Abschnitt 32 sind vorzugsweise parallel oder kollinear angeordnet, wobei letzteren Fall das in den Figuren 1, 2 und 5 gezeigte Pumpmodul 1 darstellt.

Durch eine entlang ihrer Drehachse axial beweglich gelagerte Taumelvorrichtung 41 oder eine Taumelvorrichtung 41, die zumindest in ihren die Membran 4 quetschenden Bereichen elastisch nachfedernd ausgebildet ist, lässt sich das gleichzeitige Verschließen von Pumpkanaleingang 6 und Pumpkanalausgang 7 wie auch der Pumpvorgang insgesamt verbessern. Ein ausreichender Anpressdruck kann in diesem Falle durch eine axiale Verschiebung oder Verformung der Taumelvorrichtung erreicht werden. Insbesondere kann die axiale Lagerung und/oder elastisch nachferdernde Ausbildung der Taumelvorrichtung 41 gewährleisten, dass die Taumelvorrichtung 41 auch während eines Pumpzyklusses durch eine axiale Bewegung die Membran 4 in jeder Phase des Zyklusses ausreichend andrückt, beispielsweise auch, wenn die Taumelvorrichtung 41 ein Lücke aufweist, wie in Fig. 3 gezeigt.

Die Taumelvorrichtung 41 kann zum Zweck der axialen Verschiebung axial vorgespannt sein.

Die Figuren 8 und 10 zeigen eine dritte Ausführungsform eines erfindungsgemäßen Pumpmoduls 1. Im Gegensatz zur ersten Ausführungsform, in der die Membran 4 nicht über das Deckelteil 3 hinausragt, ist die Membran 4 mit einem Wulst ausgebildet. Im nicht deformierten Zustand ist die der Rinne 23 zugewandte Unterseite der Membran 4 so ausgeformt, dass ein sicherer Verschluss des Pumpkanals 5 möglich ist. In diesem Ausführungsbeispiel ist die Unterseite eben. Die Oberseite der Membran 4 ist wulstförmig ausgebildet und ragt über das Deckteil 3 hinaus. Durch beispielsweise eine wie in Figur 9 gezeigte Taumelvorrichtung 41 kann die Membran 4 durch Druck der Taumelvorrichtung 41 auf einen Abschnitt des wulstförmigen Bereichess der Membran 4 in einem Abschnitt den Pumpkanal 5 fluiddicht verschließend an die Oberfläche der Rinne 23 gepresst werden, wodurch bei taumelnder Bewegung der Taumelvorrichtung 41 um eine Taumelachse 67 ein Fluid durch den Pumpkanal förderbar ist. Die der Membran 4 zugewandte Unterseite einer Basis 62 der Taumelvorrichtung 41 kann aufgrund der wulstförmigen Membran 4 eben ausgebildet sein, wie in Fig. 10 gezeigt. Fig. 10 zeigt die Membran 4 mit einem Abschnitt, der nicht deformiert ist, und einen Abschnitt, der durch die Taumelvorrichtung 41 deformiert ist und den Pumpkanal 5 verschließt. In ihrer übrigen Ausgestaltung entspricht die dritte Ausführungsform der ersten Ausführungsform des Pumpmoduls 1.

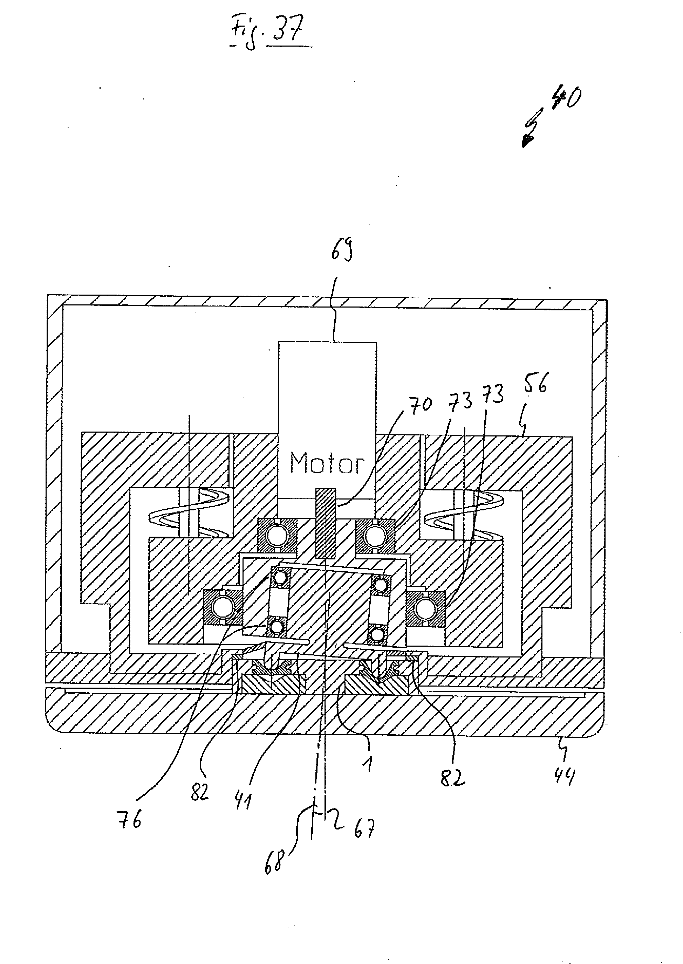

Vorteilhaft einer solchen flachen Ausführungsform einer Taumelvorrichtung 41 ist, dass die Taumelvorrichtung 41 und auch die Antriebseinheit der Taumelvorrichtung auf laterale Toleranzen unempfindlich werden. Des Weiteren kann die Taumelvorrichtung 41 einfach und definiert als semirigide oder elastische Scheibe ausgeführt werden, siehe Fig. 37. Auf diese Weise kann eine Eigenelastizität der Taumelscheibe 41 realisiert werden, um einen definierten Anpressdruck der Taumelscheibe auf die Membran zu erzielen. Axiale Toleranzen können auf diese Weise ausgeglichen werden.

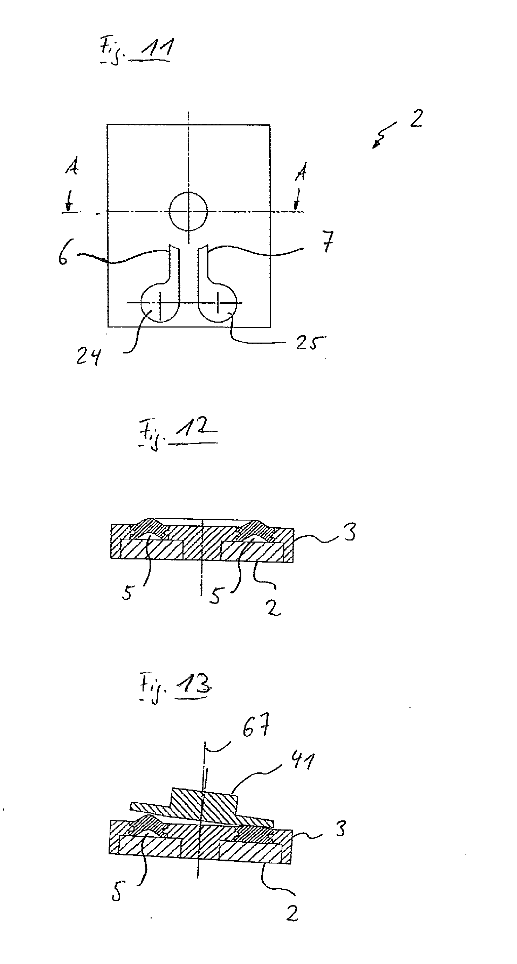

Die Figuren 11 bis 13 zeigen eine vierte Ausführungsform eines erfindungsgemäßen Pumpmoduls 1. Abweichend zur ersten Ausführungsform ist die bogenförmige Membran 4 nicht flach, sondern an Ihrer Oberseite entsprechend der dritten Ausführungsform mit einem Wulst ausgebildet. Die Unterseite der Membran 4 ist im nicht deformierten Zustand nach Innen gewölbt. Das Basisteil 2 ist, abgesehen von dem Pumpkanaleingang 6 und Pumpkanalausgang 7, die als nutförmig Einschnitte ausgebildet sind, an seiner der Unterseite der Membran 4 gegenüberliegenden Oberseite eben ausgebildet. Die nach Innen gewölbte Oberfläche der Unterseite der Membran 4 und die ebene Oberfläche des Basisteils 2 bilden einen Pumpkanal 5, durch den ein Fluid förderbar ist. Durch beispielsweise eine wie in Figur 9 gezeigte Taumelvorrichtung 41 kann die Membran 4 in einem Abschnitt den Pumpkanal 5 fluiddicht verschließend an die Oberfläche des Basisteils 2 gepresst werden. Fig. 13 zeigt die Membran 4 mit einem Abschnitt, der nicht deformiert ist, und einen Abschnitt, der durch die Taumelvorrichtung 41 deformiert ist und den Pumpkanal 5 verschließt. In ihrer übrigen Ausgestaltung entspricht die vierte Ausführungsform der ersten Ausführungsform des Pumpmoduls 1.

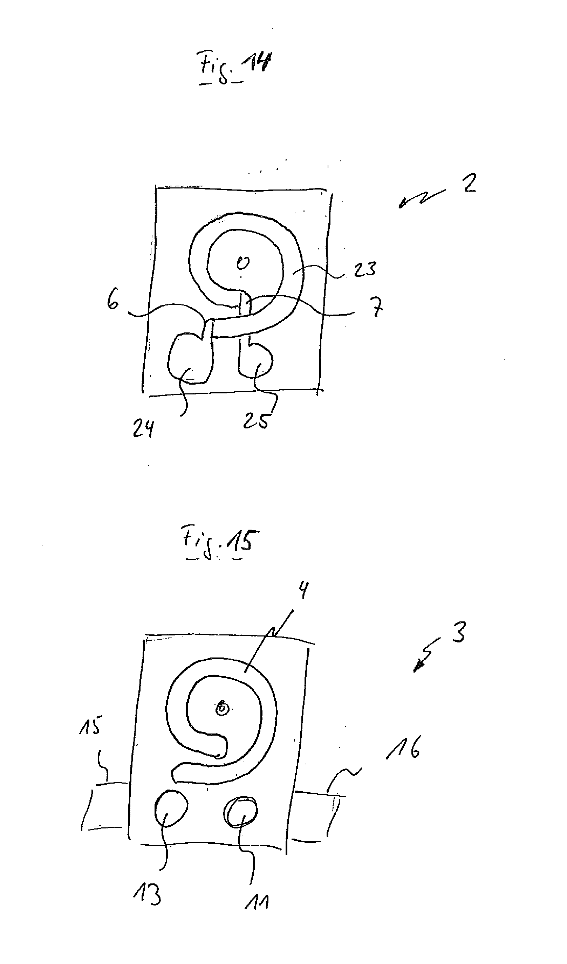

Die Figuren 14 und 15 zeigen eine fünfte Ausführungsform eines erfindungsgemäßen Pumpmoduls 1. Im Gegensatz zu der ersten Ausführungsform ist der Pumpkanal 5 zwischen Pumpkanaleingang 6 und Pumpkanalausgang 7 spiralförmig ausgebildet, wobei sich der Pumpkanal 5 in einem Abschnitt zwischen Pumpkanaleingang 6 und Pumpkanalausgang 7 radial überlappt, d.h., der Pumpkanal 5 überdeckt einen Winkelbereich von über 360°. Die der spiralförmige Pumpkanal 5 ist vorzugsweise in einer Ebene senkrecht zur Taumelachse ausgebildet. Im Überlappungsbereich geht der spiralförmige Abschnitt des Pumpkanals 5 zum Pumpkanaleingang 6 hin sowie auch zum Pumpkanalausgang 7 hin jeweils in einen geraden Abschnitt über, wobei diese beiden geraden Abschnitte parallel zueinander angeordnet sind. Als eine Alternative kann der Pumpkanal auch rein spiralförmig ausgebildet sein. Ansonsten entspricht diese Ausführungsform des Pumpmoduls 1 der ersten Ausführungsform.

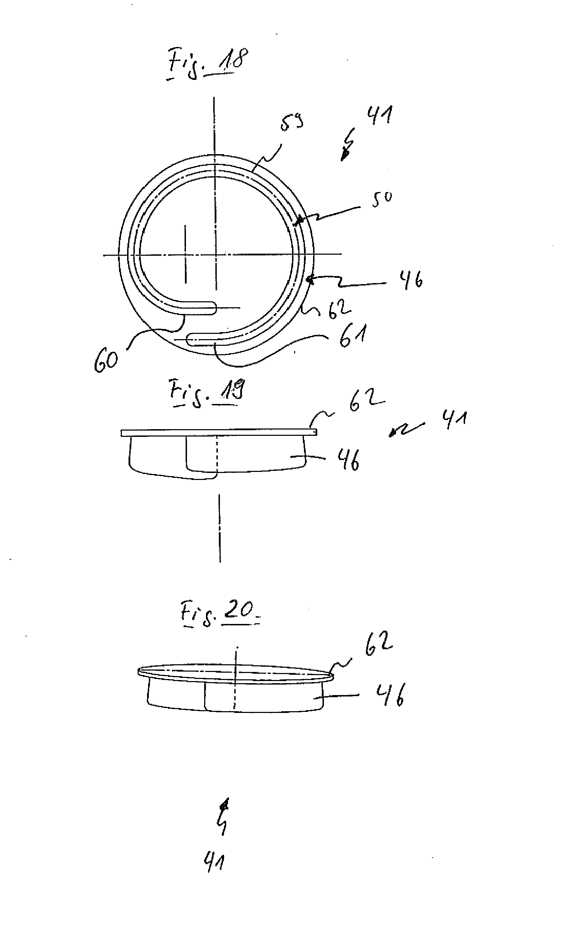

Ein spiralförmiger Abschnitt im Pumpkanal 5 ermöglicht es, Pumpkanaleingang 6 und Pumpkanalausgang 7 versetzt zueinander anzuordnen, so dass sich der Pumpkanal 5, wie in den Figuren gezeigt, in einem Abschnitt radial überlappen kann. Die Überlappung ermöglicht es, mittels einer Taumelvorrichtung 41 zu gewährleisten, dass der Pumpkanaleingang 6 und der Pumpkanalausgang 7 im Rahmen der Taumelbewegung sicher verschlossen werden können, so dass ein Zustand, an dem der Pumpkanal 5 zwischen Pumpkanalausgang 6 und Pumpkanalausgang 7 für einen Moment geöffnet ist, vermieden werden kann. Des Weiteren ermöglicht die Überlappung des Pumpkanals 5, während des Taumelvorgangs innerhalb des Pumpkanals 5 eine Kompression des geförderten Fluids zu erzeugen, vorzugsweise in dem beginnend mit dem Pumpkanaleingang 6 der Pumpkanal 5 lokal verschlossen wird, während im Abschnitt des Pumpkanalausgangs 7 die umlaufende Verformung der Membran 4 noch nicht den Pumpkanalausgang 7 erreicht hat. Bei einer weiterlaufenden Taumelbewegung wird das Volumen zwischen diesen beiden abgedichteten Bereichen des Pumpkanals 5 verkleinert, da die vom Zentrum (das vorzugsweise auf der Taumelachse liegt) entfernter liegende, den Pumpkanal 5 abdichtende lokale Verformung einen größeren Weg zurücklegt als die innen liegende Verformung. Die Kompressionsphase endet, wenn die innen liegende Verformung den Pumpkanalausgang 7 erreicht. Eine solche Kompression lässt sich mit der in den Figuren 18 bis 20 gezeigten Taumelvorrichtung 41, oder, wenn die Membran 4 mit einem entsprechenden Wulst ausgebildet ist, mit einer Taumelvorrichtung wie in Fig. 9 gezeigt, umsetzen.

Die Figuren 16 und 17 zeigen eine sechste Ausführungsform eines erfindungsgemäßen Pumpmoduls 1. In Abweichung zur ersten Ausführungsform umfasst der Pumpkanal 5 zwei kreisbogenförmige Pumpkanalabschnitte, die durch zwei die Membran 4 bildende kreisbogenförmige Membranabschnitte 401 und 402 und durch zwei kreisbogenförmige Rinnenabschnitte 231 und 232 im Basisteil 2 gebildet werden, wobei der erste Pumpkanalabschnitt in einem ersten Radius zu einem Zentrum 403 (das vorzugsweise auf der Taumelachse liegt) angeordnet ist und der zweite Pumpkanalabschnitt zu einem sich vom ersten unterscheidenden zweiten, hier größeren Radius vom Zentrum 403 angeordnet ist. Der erste Pumpkanalabschnitt erstreckt sich über einen Winkelbereich von vorzugsweise mindestens180° bis höchstens 355°, der zweite Pumpkanalabschnitt erstreckt sich vorzugsweise über einen Winkelbereich von mindestens 20°. Der erste Pumpkanalabschnitt und der zweite Pumpkanalabschnitt überlappen sich vorzugsweise mindestens in einem Winkelbereich von 10°.

Der Pumpkanaleingang 6 ist an einem Ende des zweiten, äußeren Rinnenabschnitts 232 angeordnet, der Pumpkanalausgang 7 an einem Ende des ersten, inneren Rinnenabschnitts 231. Der zweite Pumpkanalabschnitt/der zweite Rinnenabschnitt 232 ist mit dem ersten Pumpkanalabschnitt/dem ersten Rinnenabschnitt 231 über einen kurzen Überleitkanalabschnitt 233 verbunden. Der Überleitkanalabschnitt 233 führt von einer im vom Pumpkanaleingang 6 entfernten Bereich des zweiten Pumpkanalabschnitts liegenden Stelle zum dem Pumpkanalausgang 7 gegenüberliegenden Ende des ersten Pumpkanalabschnitts. Dadurch, dass der Überleitkanalabschnitt 233 vor dem Pumpkanaleingang 6 gegenüberliegenden Ende liegt, wird der Pumpkanal 5 an der Stelle des Überleitkanalabschnitts 233 in einen ersten Pumpkanalbereich (Beginn des Überleitkanalabschnitts 233 bis Pumpkanalausgang 7) und einen zweiten Pumpkanalbereich (Beginn des Überleitkanalabschnitts 233 bis zu dem Pumpkanaleingang 6 gegenüberliegendem Ende des zweiten Pumpkanalabschnitts) aufgespalten. Im ersten Pumpkanalbereich wird das Fluid zum Pumpkanalausgang 7 gefördert. Der zweite Pumpkanalbereich, der eine "Sackgasse" für das Fluid bildet, wird in diesem Ausführungsbeispiel für den Druckausgleich verwendet.

Der Druckausgleich geschieht wie folgt: In einer sich periodisch wiederholenden Phase des Taumelvorganges wird sowohl der Bereich des Pumpkanaleingangs 6 im äußeren zweiten Pumpkanalabschnitt als auch ein vor dem Pumpkanalausgang 7 liegender Bereich im inneren ersten Pumpkanalabschnitt des Pumpkanals 5 gleichzeitig abgedichtet. Bei fortschreitender Taumelbewegung, bei der gezeigten Ausführungsform gegen den Uhrzeigersinn, folgt eine Phase der Kompression, da der Radius des inneren ersten Pumpkanalabschnitts kleiner ist als der Radius des äußeren zweiten Pumpkanalabschnitts. Ein Überdruck wird durch den zweiten Pumpkanalbereich zwischen Überleitkanalabschnitt und dem Pumpkanaleingang 6 abgewandten Ende des zweiten Pumpkanalabschnitts aufgrund der Flexibilität der Membran 4 zumindest teilweise durch Deformierung der Membran 4 kompensiert. Bei fortschreitender Taumelbewegung wird schließlich der Überleitkanalabschnitt 233 überschritten, die Verformung der Membran 4 setzt sich im ersten Pumpkanalabschnitt fort, so dass sich ein in dem zweiten Pumpkanalbereich aufgebauter Überdruck abbauen kann. Vorzugsweise wird beim Erreichen des Überleitkanalabschnitts 233 sowohl im Bereich des Eingang als auch des Ausgangs der Überleitkanalabschnitts 233 die Membran 4 gleichzeitig dichtend gequetscht, um einen ungewünschten Rückfluss des Fluids bei dem Übergang der durch die Taumelbewegung verursachten Verformung der Membran 4 vom zweiten Pumpkanalabschnitt auf den ersten Pumpkanalabschnitt zu vermeiden.

Die Membran 4 kann als eine Variante in dem zweiten Pumpkanalbereich mit einer anderen Materialstärke und/oder aus einem anderen Material ausgebildet sein, was es ermöglicht, den Druckausgleich zu optimieren.

Der Überleitkanalabschnitt 233 ist in einer Ebene des Basisteils 2 ausgebildet, die in der Ebene liegt, in denen sich die Rinne 23 befindet. Vom Pumpkanalausgang 7 führt ein Zuführkanalabschnitt 234 in einer Ebene, die Unterhalb der Ebene liegt, in der sich die Rinne 23 befindet, zum zweiten Anschluss 16. Andere Ausbildungen des Überleitkanalabschnitts 233 oder des Zuführkanalabschnitts 234 sind ebenfalls möglich.

In einer alternativen Variante kann der erste und/oder der zweite Pumpkanalabschnitt anstatt kreisbogenförmig auch spiralförmig ausgebildet sein.

Als Taumelvorrichtungen eignen sich sowohl Taumelvorrichtungen 41 mit Steg, oder auch Taumelvorrichtungen 41 mit einer ebenen Kontaktfläche, falls die Membran 4 wulstförmig ausgebildet sein sollte.

Gemäß der sechsten Ausführungsform eines Pumpmoduls 1 ist die Druckmesskammer 10 im Bereich des Pumpkanals 5 zwischen Pumpkanaleingang 6 und Pumpkanalausgang 7 ausgebildet, hier im ersten inneren Pumpkanalabschnitt. Der Pumpkanal 5 dient als Messkammer. Der flexible Wandabschnitt 11 der Druckmesskammer 10 zur Druckmessung bildet einen Teil der Wand des Pumpkanals 5. Der flexible Wandabschnitt 11 liegt der Membran 4 gegenüber.

Die Druckmessung eines Fluids innerhalb des Pumpkanals 5 ermöglicht die Druckmessung sowohl stromabwärts als auch stromaufwärts, sowie im Zustand, in dem Pumpkanaleingang 6 und Pumpkanalausgang 7 gleichzeitig verschlossen sind. Dadurch lassen sich beispielsweise Okklusionen, Schlauchfehler oder leere Beutel erkennen lassen. Des Weiteren ermöglicht eine solche Druckmessung, Luftbläschen im Fluid zu erkennen, insbesondere Luftbläschen in der Größenordnung des Pumpkanals 5 oder größer. Auch das Füllvolumen eine Fluids lässt sich bei verschlossenem Pumpkanalein- und Ausgang in dem Pumpkanal 5 ermitteln.

Alternativ kann die Messkammer 10 abgetrennt vom Pumpkanal 5 ausgebildet sein und mit dem Pumpkanal 5 über eine Zuleitung verbunden sein.

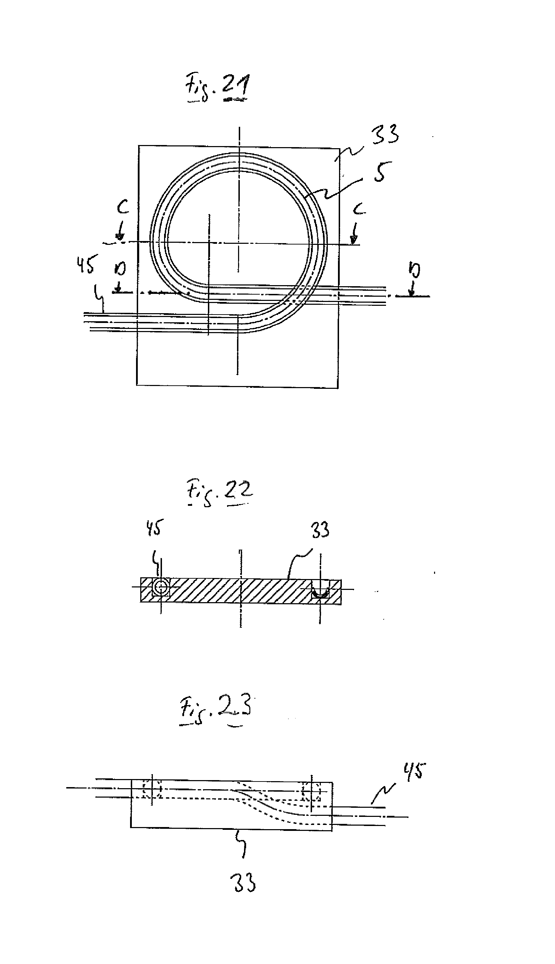

In einer alternativen siebten Ausführungsform, die die Figuren 21 bis 23 in verschiedenen Ansichten zeigt, wird der Pumpkanal 5 durch den Abschnitt eines elastisch deformierbaren Schlauches 45 ausgebildet. Der Pumpkanal 5 hat einen spiralförmigen Abschnitt, der beidseitig in jeweils einen geradlinigen Abschnitt übergeht, wobei die beiden geradlinigen Abschnitte parallel zueinander versetzt angeordnet sind. Der innen liegende geradlinige Abschnitt des Schlauches 45 taucht unter den spiralförmigen Abschnitt hindurch, siehe Fig. 23. Der Schlauch 45 ist in diesem Ausführungsbeispiel in einem nutförmigen Kanal eines starren Trägers 33 angeordnet, wodurch der Schlauch 45 in seiner Form fixiert ist. Als Schlauch 45 eignen sich beispielsweise die üblicherweise für peristaltische Schlauchpumpen verwendeten flexiblen, elastisch deformierbaren Schläuche.

Die Pumpkanäle 5 der in den Ausführungsbeispielen gezeigten Pumpmodule 1 weisen einen Querschnitt auf, der in dem Abschnitt zwischen Pumpkanaleingang 6 und Pumpkanalausgang 7 einen Wert im Bereich von 0,1 mm² ≤ Q ≥ 10 mm², vorzugsweise einen Wert im Bereich von 0,5 mm² ≤ Q ≤ 2 mm² aufweist. Des Weiteren weist das Volumen VS des Pumpkanals 5 in dem Abschnitt zwischen Pumpkanaleingang 6 und Pumpkanalausgang 7 einen Wert im Bereich von 1 µl ≤ VS ≤ 500 µl, vorzugsweise einen Wert im Bereich von 10 µl ≤ VS ≤ 100 µl auf. Das Basisteil 2 und das mit dem Basisteil 2 fest verbundene Deckelteil 3 weisen gemeinsam eine Länge und Breite von höchstens 100 mm, vorzugsweise höchstens 50 mm, besonders bevorzugt von höchstens 25 mm auf, und eine Dicke von höchstens 20 mm, vorzugsweise höchstens 10 mm, besonders bevorzugt von höchstens 5 mm auf.

Das Taumelpumpenbasismodul 40 umfasst neben einem Taumelpumpenantrieb 43 eine bereits erwähnte Taumelvorrichtung 41, mit der die Membran 4 gequetscht werden kann. Dabei ist die Taumelvorrichtung 41 in ihrer Geometrie an den Pumpkanal 5 angepasst. In diesem Falle umfasst die Taumelvorrichtung 41 einen in eine Taumelbewegung versetzbaren, linienförmigen und zumindest abschnittsweise gekrümmten Steg 46 zum taumelnden Verformen der Membran 4, wobei der Steg 46 wie die Pumpkanäle 5 der Pumpmodule 1 der beschriebenen Ausführungsformen abweichend von einer reinen Kreisbogenform ausgebildet ist.

Alternativ kann der Steg 46 auch kreisbogenförmig ausgebildet sein, wobei in diesem Falle vorzugsweise die Taumelvorrichtung 41 axial beweglich gelagert ist oder die Taumelvorrichtung 41 nachfedernd ausgebildet ist, wie beispielsweise in Fig. 37 gezeigt.

Der Steg 46 ist an einer scheibenförmigen Basis 62 der auf diese Weise als Taumelscheibe ausgebildeten Taumelvorrichtung 41 befestigt. Alternativ kann die Taumelvorrichtung 41 ohne Steg mit einer ebenen Druckfläche ausgebildet sein, falls das Pumpmodul 1 eine Membran 4 aufweist, die einen Wulst umfasst.

Gemäß einer ersten Ausführungsform einer Taumelvorrichtung 41 umfasst der Steg 46 einen kreisförmigen Abschnitt 47, einen ersten geraden Abschnitt 48 und einen zweiten geraden Abschnitt 49, wobei der kreisförmige Abschnitt 47 zwischen dem ersten geraden Abschnitt 48 und dem zweiten geraden Abschnitt 49 angeordnet ist, siehe Fig. 3. Der Verlauf des Steges 46 entspricht dem Verlauf des Pumpkanals 5 der ersten Ausführungsform des Pumpenmoduls 1, das in den Figuren 1,2, 4 und 5 gezeigt ist. Der Steg 46 ist nicht vollständig umlaufend ausgebildet, sondern in einem Abschnitt zwischen dem ersten geraden Abschnitt 48 und dem zweiten geraden Abschnitt 49 ausgespart. Die Aussparung dient zur Überbrückung des Stegs 26 des Pumpenmoduls 1, der den Pumpkanaleingang 6 vom Pumpkanalausgang 7 trennt.

Im ersten geraden Abschnitt 48 und im zweiten geraden Abschnitt 49 läuft der Steg 46 jeweils in Form einer abgerundeten Zunge aus. Diese Zungenform ermöglicht ein federndes Nachgeben der Endabschnitte des ersten geraden Abschnitts 48 und des zweiten geraden Abschnitts 49. Damit kann die Belastung der Membran 4 im Bereich des Pumpkanaleingangs 6 und des Pumpkanalausgangs 7 durch die Taumelvorrichtung 41 reduziert werden.

Der Steg 46 kann grundsätzlich mit einer einheitlichen Steghöhe ausgebildet sein, worunter hier zu verstehen ist, dass der dem Pumpmodul 1 entgegenzurichtende Stegrand des Steges 46 in einer Ebene liegt. Gemäß der ersten Ausführungsform der Taumelvorrichtung 41 ist der Steg 46 mit einer variierenden Steghöhe ausgebildet, d.h., der Stegrand des Steges 46 liegt nicht in einer gemeinsamen Ebene. Im Bereich des ersten geraden Abschnitts 48 und des zweiten geraden Abschnitts 49 verringert sich die Höhe des Steges 46 bogenförmig (eine gestrichelte Linie 58 in Figur 3 zeigt den Verlauf des Steges bei konstanter Höhe). Dies ermöglicht es, dass die Taumelvorrichtung 41 während der Taumelbewegung gleichzeitig den Pumpkanaleingang 6 und den Pumpkanalausgang 7 eines Pumpkanals 5 eines Pumpmoduls 1 gemäß der ersten Ausführungsform sicher abquetscht, so dass Rückflussprobleme vermieden werden. Die Taumelvorrichtung 41 ist vorzugsweise axial, beispielsweise federnd, gelagert, um durch eine axiale Bewegung in Richtung Pumpmodul 1 ein sicheres Abdichten von Pumpkanaleingang 6 und Pumpkanalausgang 7 zu gewährleisten.

Die Figuren 18 und 19 zeigen zwei Ansichten einer bereits erwähnten dritten Ausführungsform einer Taumelvorrichtung 41. In dieser Ausführungsform umfasst der Steg 46 der Taumelvorrichtung 41 einen spiralförmigen Abschnitt 59, an den sich beidseitig jeweils ein geradliniger erster und zweiter Abschnitt 60, 61 anschließt, wobei die geradlinigen Abschnitte 60, 61 parallel zueinander angeordnet sind, so dass sich der Steg 46 überlappt. Der Verlauf des Steges 46 entspricht dem Verlauf des Pumpkanals 5 der fünften Ausführungsform des Pumpmoduls 1, siehe Figuren 14, 15. Der Steg 46 weist in einem ersten Abschnitt des spiralförmigen Abschnitts eine größere Höhe auf als in einem zweiten, von Zentrum der Spirale entfernter liegenden zweiten Abschnitt des spiralförmigen Abschnitts, siehe Fig. 19. In diesem Ausführungsbeispiel erhöht sich die Steghöhe mit abnehmendem Radius der Spirale. An den spiralförmigen Abschnitt schließen sich die geradlinige Abschnitt 60, 61 an. Dadurch, dass der Steg 46 in einem zum Zentrum näher liegenden Bereich erhöht ist, kann gewährleistet werden, dass der Pumpkanal 5 immer sicher abgequetscht ist.

Die Figur 20 zeigt eine Variante der in den Figuren 18 und 19 gezeigten zweiten Ausführungsform der Taumelvorrichtung 41.

Alternativ oder ergänzend ist es möglich, den Pumpkanal 5 des Pumpmoduls 1 mit variierender Steigung auszubilden, oder die Dicke der Membran 4 oder deren Wulsthöhe in Abhängigkeit vom Taumelzentrum zu variieren.

Der Steg 46 der Taumelvorrichtung 41 ist vorzugsweise ein starrer Körper. Als Materialien eignen sich insbesondere Kunststoffe oder auch Metalle. Vorzugsweise ist die Steg 46 ein Spritzgussteil. Der Steg 46 kann insbesondere einstückig mit der Basis 62 als Taumelscheibe ausgebildet sein. Des Weiteren kann der Steg aus einem starren Material, aber beispielsweise aber auch aus einem halbstarren Material ausgebildet sein. Durch die Eigenelastizität des Steges 46 kann dieser sich besser dem Membran- oder Rillenprofil anpassen, was auch bei radialen und/oder axialen Toleranzkompensationen vorteilhaft sein kann.

In der flachen Ausführungsform der als Taumelscheibe ausgebildeten Taumelvorrichtung 41 nach Fig. 9 ist die Basis 62 vorzugsweise ein halbstarrer Körper. Durch deren Eigenelastizität kann eine axiale gefederte Beweglichkeit der Taumelscheibe realisiert werden, wodurch der Anpressdruck definiert werden kann, um ein periodisch umlaufendes, sicheres Abquetschen des Pumpkanals 5 zu erreichen und insbesondere den gleichzeitigen Verschluss von Eingang und Ausgang zu gewährleisten. Außerdem lassen sich axiale Toleranzen zwischen Pumpenbasismodul und Pumpmodul damit kompensieren.

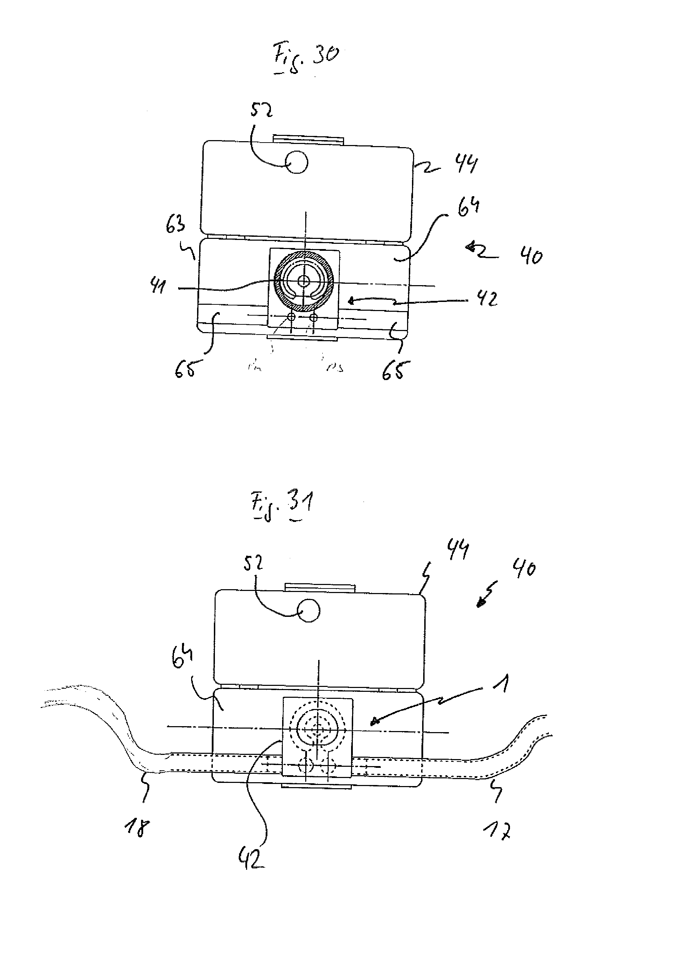

Die Figuren 30 bis 37 zeigen verschiedene Taumelpumpensysteme, die ein Taumelpumpenbasismodul 40 und ein Pumpenmodul 1 umfassen.

Ein Taumelpumpenbasismodul 40 umfasst einen Taumelpumpenantrieb 43 und eine Taumelpumpenvorrichtung 41, siehe beispielsweise Figur 33. Mittels des Taumelpumpenantriebs 43 lässt sich die Taumelpumpenvorrichtung 41 zur Ausführung einer taumelnden Bewegung antreiben. Des Weiteren umfasst das Taumelpumpenbasismodul 40 ein Gehäuse, in dem der Taumelpumpenantrieb 43 und die Taumelpumpenvorrichtung 41 angeordnet sind. Das Gehäuse umfasst einen Gehäusedeckel 63 und einen Gehäuseboden 64.

Zur Aufnahme eines Pumpenmoduls 1 umfasst das Taumelpumpenbasismodul 40 eine Aufnahme 42, siehe auch Figuren 30 und 31. Die Aufnahme 42 ist derart ausgebildet, dass in die Aufnahme 42 ein erfindungsgemäßes Pumpenmodul 1 händisch eingesetzt oder eingelegt und aus der Aufnahme 42 händisch entnommen werden kann. Händisch heißt in diesem Zusammenhang, dass eine Zuhilfenahme von Werkzeug für das Einsetzen oder Entnehmen des Pumpmoduls 1 nicht notwendig ist.

In diesem Ausführungsbeispiel ist die Aufnahme 42 in Form einer Mulde oder Wanne ausgebildet. Die Aufnahme 42 korrespondiert zu der Außenform des Pumpenmoduls 1, so dass das Pumpenmodul 1 formschlüssig von der Aufnahme 42 aufgenommen werden kann. Dies ist vorteilhaft für eine korrekte Positionierung des Pumpenmoduls 1 bezüglich der Taumelvorrichtung 41. Entsprechend der Quaderform der Pumpenmodule 1 ist in diesem Falle die Aufnahme 42 quaderförmig ausgebildet. Andere Formen für das Pumpenmodul 1 und für die Aufnahme 42 sind selbstverständlich ebenfalls möglich.

Am Boden der Aufnahme 42 umfasst die Aufnahme 42 eine Aussparung. Im Bereich der Aussparung ist die Taumelvorrichtung 41 angeordnet, so dass die Taumelvorrichtung 41 in Wirkverbindung mit einem in die Aufnahme 42 eingesetzten Pumpmodul 1 treten kann. Vorzugsweise ist der Spalt zwischen Aufnahme 42 und Taumelvorrichtung 41 fluiddicht abgedichtet, beispielsweise mittels einer flexiblen Membran 65, um einen ungewünschten Eintritt eines Fluids, das beispielsweise durch unsachgemäße Behandlung oder eines fehlerhaften Pumpenmoduls 1 in die Aufnahme 42 gelangt ist, in das Innere des Gehäuses des Pumpenbasismoduls 40 zu vermeiden.

Der Gehäuseboden 64 umfasst zusätzlich zur Aufnahme 42 zwei von der Aufnahme 42 abgehende nutförmige Kanäle 65. In die nutförmige Kanäle 65 können vom Pumpenmodul 1 beidseitig abgehende Schlauchabschnitte eingelegt werden.

Durch eine zumindest teilweise asymmetrische Ausbildung der Form des Pumpenmoduls 1 und entsprechend der Aufnahme 42 kann gewährleistet werden, dass das Pumpenmodul 1 nur mit einer Orientierung in die Aufnahme 42 eingesetzt werden kann. Auf diese Weise lässt sich das Risiko eines falschen Gebrauches mindern. In diesem Falle sind die Schlauchstutzen 15, 16 des Pumpenmoduls 1 nicht mittig, sondern versetzt zur Mitte des Pumpenmoduls 1 angeordnet.

Die Aufnahme 42 kann ein Teil des Gehäuses sein. Im Ausführungsbeispiel wird die Aufnahme 42 durch den Gehäuseboden 64 ausgebildet.

Das Taumelpumpenbasismodul 40 weist ferner eine Sicherungsvorrichtung auf, mittels der das Pumpenmodul 1 in seiner Position in der Aufnahme 42 sicherbar ist. Die Sicherungsvorrichtung ist händisch, also ohne Zuhilfenahme von Werkzeug, betätigbar, was eine einfache und unkomplizierte Bedienung ermöglicht.

In diesem Ausführungsbeispiel umfasst die Sicherungsvorrichtung einen Deckel 44. Der Deckel 44 ist klappbar mit dem Gehäuse, hier mit dem Gehäuseboden 64, verbunden, beispielsweise mittels eines Scharniers (nicht dargestellt). Mit dem Deckel 44 ist die Aufnahme 44 verschließbar. Im geschlossenen Zustand des Deckels 44 sichert der Deckel 44 durch Formschluss das Pumpenmodul 1 in seiner Position in der Aufnahme 42, siehe beispielsweise Figuren 22 und 23. Im geöffneten Zustand des Deckels 44 ist das Pumpenmodul 1 aus der Aufnahme 42 händisch entnehmbar.

Zum Sichern des Deckels 44 in seiner geschlossenen Position können bekannte Vorrichtungen verwendet werden. Beispielsweise können Schnapp-, Rast- oder andere Verriegelungsmechanismen eingesetzt werden. Der geschlossene Zustand des Deckels kann auch durch eine automatisch gesteuerte (eventuell zusätzliche) Verriegelung gesichert werden, um beispielsweise zu verhindern, dass der Deckel 44 während des Betriebes der Pumpe geöffnet wird.

Des Weiteren umfasst das Taumelpumpenbasismodul 40 eine Ventiibetätigungsvorrichtung. Die Ventilbetätigungsvorrichtung ist derart ausgebildet, dass diese beim Einsetzen oder Einlegen des Pumpenmoduls 1 oder nach dem Einsetzen oder Einlegen des Pumpendmoduls 1 den ersten flexiblen Wandabschnitt der Ventilkammer 12 des Pumpenmoduls 1 verformt und damit den Ventilkörper 14 in die Betätigungsstellung bringt.

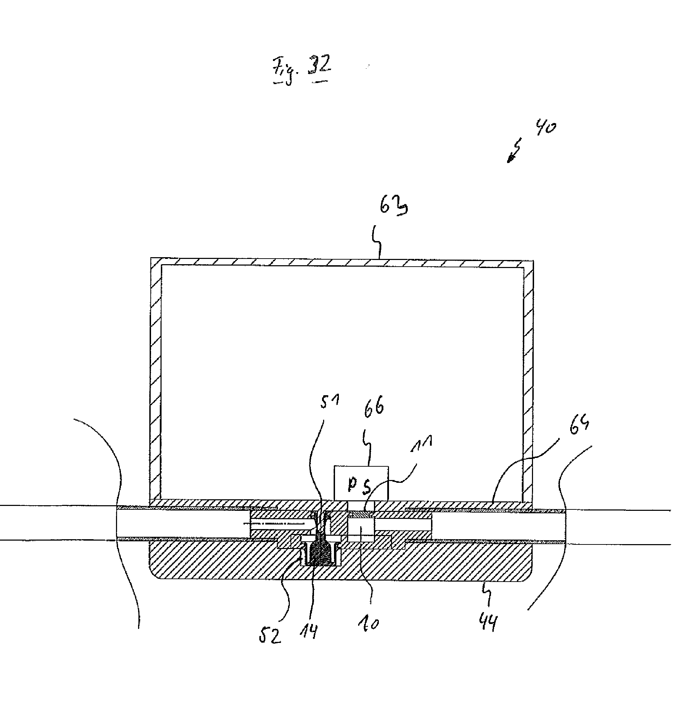

In diesem Ausführungsbeispiel wird die Ventilbetätigungsvorrichtung durch einen starren, hervorstehenden Stift 51, siehe Figur 32, ausgebildet. Der Stift 51 ist unbeweglich am Boden der Aufnahme 42 angeordnet. Der Stift 51 ist zum Eingriff in die Vertiefung 21 des Pumpmoduls 1, siehe auch Fig. 24, ausgebildet. Durch Einsetzen des Pumpmoduls 1 greift der Stift in die Vertiefung 21 ein, verformt den ersten flexiblen Wandabschnitt 13 und bringt auf diese Weise den Ventilkörper 14 in die Betätigungsstellung. Je nach Ausbildung des ersten flexiblen Wandabschnitts 13 und der Vertiefung 21 kann die Ventilbetätigungsvorrichtung eine andere Form aufweisen, beispielsweise als Steg ausgebildet sein oder, wenn die Membran 13 anstatt mit einer Vertiefung oder flach mit einer Erhöhung ausgebildet ist, selbst eine Vertiefung oder Aussparung aufweisen, in die die Erhöhung der Membran 13 beim Einsetzen des Pumpmoduls 1 in Eingriff zu bringen ist.

Alternativ ist es ebenfalls möglich, an Stelle eines unbeweglichen Vorsprungs wie einen festen Stift oder einen Steg einen beweglichen, herausfahrbaren Vorsprung als Ventilbetätigungsvorrichtung vorzusehen (nicht dargestellt). Dies ermöglicht es, zu einem gewünschten Zeitpunkt den Ventilkörper 14 zum Öffnen zu betätigen, beispielsweise erst dann, wenn der Betrieb der Pumpe aufgenommen werden soll. Bei Beendigung des Pumpbetriebs kann der Vorsprung zurückgezogen und damit der Ventilkörper 14 in seine Ruhestellung zurückkehren. Der herausfahrbare und zurückfahrbare Vorsprung kann beispielsweise ebenfalls als Stift oder Steg ausgebildet sein.

Die Ventilbetätigungsvorrichtung 51 kann ergänzend als Positioniermittel für das Pumpenmodul 1 verwendet werden und als Mittel verwendet werden, um ein korrektes Einlegen des Pumpenmoduls 1 in die Aufnahme zu gewährleisten.

Das Taumelpumpenbasismodul 40 umfasst eine Aussparung 52, in die der zweite flexible Wandabschnitt 20 der Ventilkammer 12 des Pumpenmoduls 1 beim Übergang des Ventilkörpers 14 in die Betätigungsstellung ausweichen kann. In diesem Ausführungsbeispiel ist die Aussparung 52 im Deckel 44 angeordnet, siehe Figuren 30 bis 32. Damit ist es möglich, das Pumpenmodul 1 als flache und kleine Bauform auszubilden.

Des Weiteren umfasst das Taumelpumpenbasismodul 40 einen Drucksensor 66, siehe Fig. 32. Der Drucksensor 66 ist derart ausgebildet, dass er anhand der Verformung des flexiblen Wandabschnitts 11 der Druckmesskammer 10 einen Wert ermittelt, der den Druck innerhalb der Druckmesskammer 10 widerspiegelt. Derartige Drucksensoren 66 sind dem Fachmann hinlänglich bekannt. Der Drucksensor 66 ist in diesem Ausführungsbeispiel im Gehäuse des Taumelpumpenbasismoduls 40 angeordnet.

Alternativ oder zusätzlich kann der Drucksensor 66 bzw. ein weiterer Drucksensor im Kontakt mit der Membran 4 des Pumpmoduls 1 stehen, um den Druck in dem Pumpkanal 5 zu messen, wie bereits zuvor beschrieben.

Das Taumelpumpenbasismodul 40 umfasst zusätzlich eine Vorspanneinrichtung 56, siehe beispielsweise Fig. 33. Die Vorspanneinrichtung 56 ist derart ausgebildet, dass die Taumelvorrichtung 41 gegen ein in der Aufnahme 42 aufgenommenes Pumpmodul 1 federnd vorgespannt wird.

Aufgrund der federnden Vorspannung der Taumelvorrichtung 41 gegen das Pumpmodul 1 kann eine definierte Lage von Taumelvorrichtung 41 zum Pumpmodul 1und/oder ein ausreichender Anpressdruck der Taumelvorrichtung 41 auf die Membran 4 des Pumpmoduls 1 erreicht werden. Dadurch kann auch gewährleistet werden, dass beim Austausch des Pumpmoduls 1 sich die Eigenschaften der Pumpe sich nicht oder nur minimal ändern. Zum anderen können axiale Toleranzen kompensiert werden.

In diesem Ausführungsbeispiel ist die Taumelvorrichtung 41 in axialer Richtung durch die Vorspanneinrichtung 56 vorgespannt. Entsprechend ist die Taumelvorrichtung 41 in axialer Richtung, in Fig. 33 durch die Achse 67 gekennzeichnet, verschiebbar gelagert. Die Vorspanneinrichtung 56 drückt die Taumelvorrichtung 41 bis zu einem Anschlag 83 in eine Ausgangsstellung, in der die Taumelvorrichtung 41 bei noch nicht in die Aufnahme 42 eingelegtem Pumpmodul 1 in die Aufnahme 42 hineinragt. Die Achse 67 ist senkrecht zum Boden der Aufnahme 42 orientiert. Der Steg 56 der Taumelvorrichtung 41 ragt damit in die Aufnahme 42 hinein. Beim Einsetzen eines Pumpmodul 1 kommt der Steg 56 in Kontakt mir der Membran 4 des Pumpmoduls 1 und quetscht die Membran 4 an einer Quetschstelle dichtend ab. Bei vollständiger Aufnahme des Pumpmoduls 1 wird die Taumelvorrichtung 41 durch das Pumpmodul 1 gegen die Vorspannung in Richtung des Gehäuses gedrückt.

In diesem Ausführungsbeispiel umfasst die Vorspanneinrichtung 56 mehrere Federn 69, die den Taumelpumpenantrieb 43 gemeinsam mit der Taumelvorrichtung 41 vorspannen. Um zu gewährleisten, dass die Taumelvorrichtung 41 nicht oder nur geringfügig gegen die Aufnahme 42 verkippt, ist der Taumelpumpenantrieb 43, mit dem die Taumelvorrichtung 41 verbunden ist, auf mehreren Führungszapfen 70 der Vorspanneinrichtung 56 gelagert. Die Vorspanneinrichtung 56 ist fest mit dem Gehäuse des Taumelpumpenbasismoduls 40, hier dem Gehäuseboden 64, verbunden.

Gemäß einer weiteren Ausführungsform (nicht gezeigt) ist die Vorspanneinrichtung im Deckel 44 des Pumpenbasismoduls 40 integriert, Die Vorspanneinrichtung ist derart ausgebildet, dass diese bei in der Aufnahme 42 eingelegtem Pumpmodul 1 und geschlossenem Deckel 44 einen Druck auf das Pumpmodul 1 ausübt, wodurch das Pumpmodul gegen die Taumelvorrichtung 41 gedrückt wird. Bei geschlossenem Deckel 44 ist damit das Pumpmodul 1 gegen die Taumelvorrichtung 1 vorgespannt.

Eine axiale Vorspannung der Taumelvorrichtung 41 entlang der Achse 67 ermöglicht es zu dem, dass die Taumelvorrichtung 41 während des Pumpbetriebes neben der Taumelbewegung eine überlagerte Bewegung in axialer Richtung ausführen kann. Gleiches gilt, wenn das Pumpmodul 1 axial gegen die Taumelvorrichtung 41 vorgespannt ist. Dies ermöglicht für bestimmte Varianten des Pumpmoduls 1 und der Taumelvorrichtung 41 ein sicheres, periodisch umlaufendes Abquetschen des Pumpkanals 5 und insbesondere ein sicheres gleichzeitiges Verschließen von Pumpkanaleingang 6 und Pumpkanalausgang 7 des Pumpmoduls 1.

Die Taumelvorrichtung 41 umfasst des Weiteren ein Positioniermittel 57. Das Positioniermittel 57 ist derart ausgebildet, dass es die Taumelvorrichtung 41 auf die Verlängerung der Motorachse zentriert. Eine korrespondierende Aufnahme oder Aussparung des Pumpmoduls 1 ermöglicht die axiale Beweglichkeit der Taumelvorrichtung 41. In diesem Ausführungsbeispiel umfasst die Taumelvorrichtung 41 einen im Zentrum angeordneten, kuppelförmigen Vorsprung. Das Pumpmodul 1 umfasst eine korrespondierende muldenförmige Aussparung 22, in die das Positioniermittel bei in der Aufnahme 42 eingesetztem Pumpmodul eingreift. Vorzugsweise besteht kein direkter Kontakt zwischen Positioniermittel 57 und Pumpmodul 1, um die Beweglichkeit der Taumelvorrichtung zu gewährleisten und um Reibungsverluste zu vermeiden.

Mittels des Taumelpumpenantriebes 43 wird die Taumelvorrichtung 41 in eine taumelnde Bewegung versetzt. Die Figuren 33 bis 36 zeigen Taumelpumpenbasismodule 40 mit verschiedenen Ausführungsformen eines Taumelpumpenantriebs 43.

Der Taumelpumpenantrieb 43 umfasst einen Motor 69, mit dem eine Antriebswelle 70 in Drehung versetzt werden kann. Die Drehung der Antriebswelle 70 erfolgt um die Achse 67. Die Drehung wird durch einen Übertragungsmechanismus in eine Taumelbewegung der Taumelvorrichtung 41 umgesetzt.

Der Motor ist vorzugsweise ein Elektromotor, beispielsweise ein Gleichstrommotor, ein Schrittmotor oder ein Piezomotor. Als Energieversorgung für den Motor wird vorzugsweise eine oder mehrere Batterien eingesetzt (nicht gezeigt), die vom Taumelpumpenbasismodul 40 aufgenommen werden. Auch eine externe Energieversorgung ist alternativ oder zusätzlich möglich.

In einer ersten Ausführungsform, die Figur 33 darstellt, umfasst der Taumelpumpenantrieb 43 ein Übertragungselement 71. Das Übertragungselement 71 ist mittels Kugellagern 73 in einem Trägerelement 72, das auch den Motor 69 aufnimmt, drehbar gelagert. Das Übertragungselement 71 ist fest mit der Antriebswelle 70 verbunden, so dass es der Drehbewegung der Antriebswelle 70 folgt.

Das Übertragungselement 71 umfasst einen seitlichen Zapfen 74. Der Zapfen 74 ist in seiner Achse gegenüber der Achse 67 geneigt. Der Neigungswinkel des Zapfens 74 entspricht einem 90°-Winkel abzüglich eines Taumelwinkels 68. Der Taumelwinkel 68 bestimmt die Neigung der Taumelvorrichtung 41 zur Achse 67.

Auf dem Zapfen 74 ist ein Kugellager 75 angeordnet. Der Außenrand des Kugellagers 75 liegt auf dem Randbereich der Rückseite der Taumelvorrichtung 41 auf.

Die Taumelvorrichtung 41 wird durch einen innerhalb des Übertragungselementes 71 mittels Kugellagern 76 drehbar gelagerten Zentrierstiftes 77 in Position gehalten. Der Zentrierstift 77 greift hierbei lose in eine im Zentrum angeordnete Vertiefung 78 der Taumelvorrichtung 41 ein. Die Drehachse des Zentrierstiftes 77 entspricht der Achse 67. Der kugelgelagerte Zentrierstift 77 dreht nicht mit, was Reibungsverluste in der Vertiefung 78 minimiert. Die Vertiefung 78 der Taumelvorrichtung 41 ermöglicht aufgrund Ihrer Form ein ausreichendes Verkippen der Taumelvorrichtung 41 gegenüber dem Zentrierstift 77. Alternativ ist es möglich, die Taumelvorrichtung 41 fest mit dem Zentrierstift 77 zu verbinden, wobei die Taumelvorrichtung 41 gegenüber dem Zentrierstift 77 die vorgesehene Neigung einnimmt.

Eine Drehbewegung der Antriebswelle 70 führt zu einer Drehbewegung des Zapfens 77 um die Drehachse 67. Das auf dem Zapfen 77 angeordnete Kugellager 75 rollt auf dem Außenrand der Taumelvorrichtung 41 ab und versetzt die Taumelvorrichtung 41 aufgrund der Neigung des Zapfens 77 in eine Taumelbewegung.

Die Symmetrieachse der Taumelvorrichtung 41 ist um den Taumelwinkel 68 gegenüber der Drehachse 67 geneigt. Vorteilhaft bei dieser Ausführungsform ist, dass sich die Taumelvorrichtung 41, hier als Taumelscheibe ausgebildet, sehr einfach zentrieren lässt.

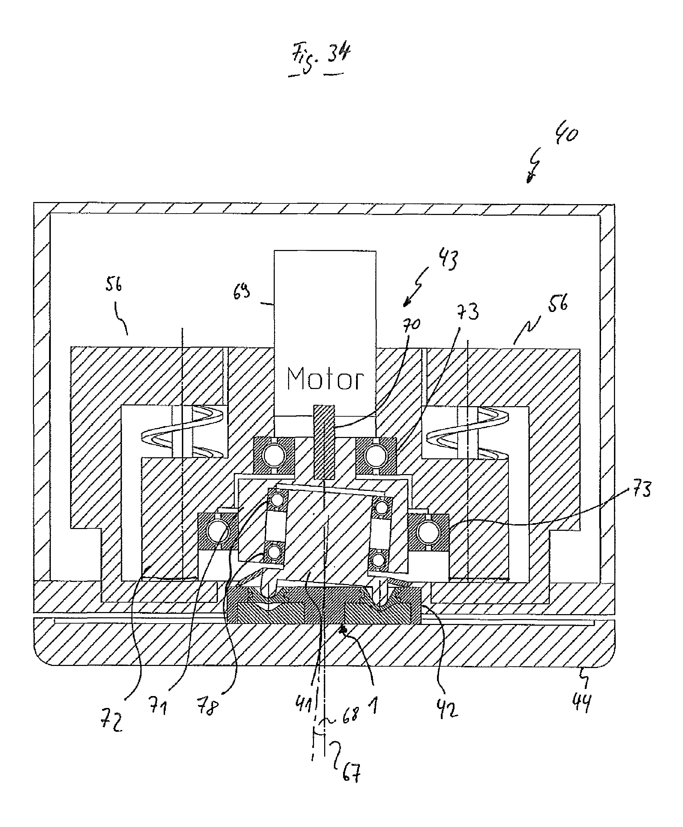

In einer Variante dieser Ausführungsform, die in Fig. 34 gezeigt ist, ist die Taumelvorrichtung 41 mittels zweier Kugellager 78, die zentrisch um die Symmetrieachse der Taumelvorrichtung 41 positioniert sind, drehbar im Übertragungselement 71 gelagert. Die beiden Kugellager 78 entkoppeln die Drehbewegung des Übertragungselementes 71 von der Taumelvorrichtung 41. Die Zentrierung der Taumelvorrichtung 41 ist in dieser Ausführungsform fest vorgegeben.

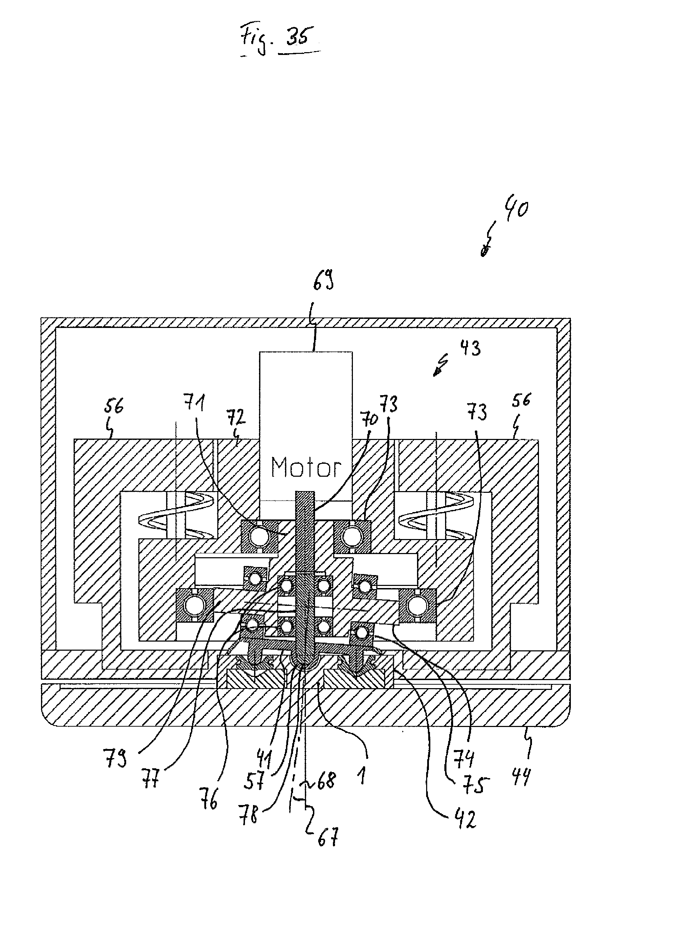

In einer anderen Variante dieser Ausführungsform, die in Fig. 35 gezeigt ist, umfasst das Übertragungselement neben dem ersten Zapfen 74 einen weiteren Zapfen 79. Der Zapfen 79 ist kollinear zum ersten Zapfen 74 gegenüberliegend auf der anderen Seite der Achse 67 angeordnet. Auf dem zweiten Zapfen 79 ist ein weiteres Kugellager 75 angeordnet. Der Außenrand des Kugellagers 75 liegt ebenfalls auf dem Randbereich der Taumelvorrichtung 41 auf. Durch eine Drehbewegung der Antriebswelle 70 rollen die beiden auf den Zapfen 74 und 9 angeordneten Kugellager 75 an gegenüberliegenden Stellen auf dem Außenrand der Taumelvorrichtung 41 ab, wodurch die Taumelvorrichtung 41 in eine Taumelbewegung versetzt wird. Im Vergleich zur in Figur 33 gezeigten Ausführungsform lässt sich auf diese Weise die Taumelbewegung stabilisieren.

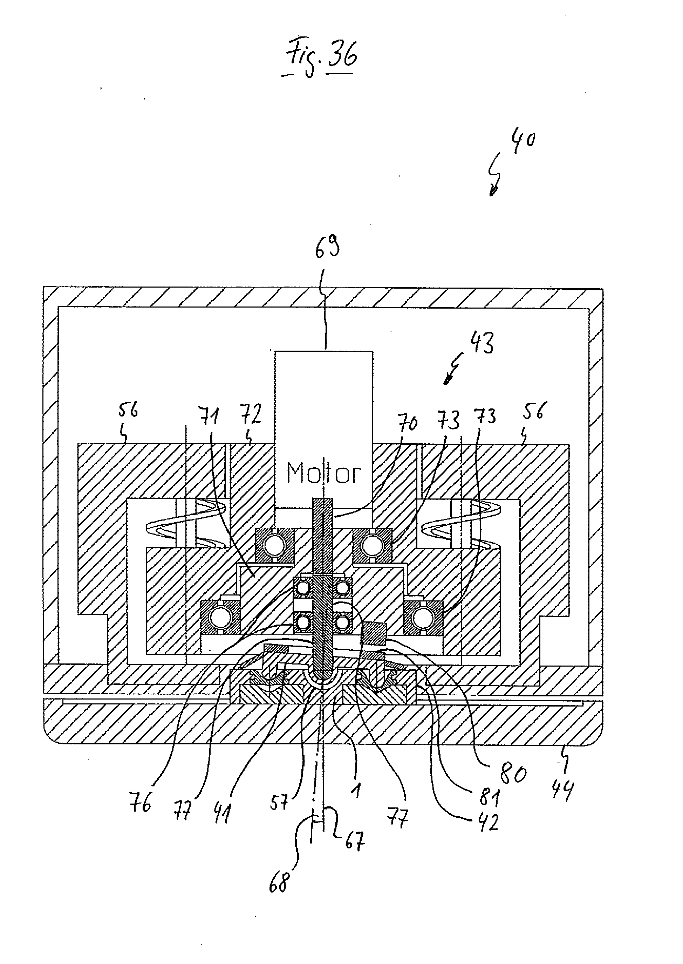

In einer anderen Variante dieser Ausführungsform, die in Fig. 36 gezeigt ist, ist der geneigte Zapfen 74 des Übertragungselementes 71 und das Kugellager 75 durch einen Magneten 80 ersetzt. Die Taumelvorrichtung 41 umfasst einen ringförmigen Magneten 81, der auf der dem Magneten 80 zugewandten Rückseite der Taumelvorrichtung 41 angeordnet ist. Der Magnet 80 des Übertragungselementes 71 ist auf einen Winkelabschnitt begrenzt. Durch Drehung des Übertragungselementes 71 überstreicht der Magnet 80 den ringförmigen Magneten 81 der Taumelvorrichtung 41. Die Magneten 80, 81 sind mit zueinander entgegengesetzten Polen angeordnet, so dass sich die Magneten 80, 81 abstoßen. Durch die Drehbewegung des Magneten 80 des Übertragungselementes 71 wird aufgrund der Abstoßwirkung der Magneten 80, 81 die Taumelvorrichtung 41 in eine Taumelbewegung versetzt. Vorteilhaft an dieser Ausführungsform ist, dass Reibungsverluste minimiert werden können. Die Zentrierung der Taumelvorrichtung wird durch den Zentrierstift 77 erreicht.

In einer weiteren Variante, die in Fig. 37 gezeigt ist, ist die Taumelvorrichtung 41 als flache, halbstarre und elastisch verformbare Taumelscheibe ausgebildet.

Dadurch, dass die Taumelvorrichtung 41 elastisch nachgeben kann, kann ein ausreichender Anpressdruck auf die Membran 4 eines eingesetzten Pumpmoduls 1 während des gesamten Pumpzyklusses gewährleistet werden. Auch können axiale Toleranzen kompensiert werden.

In diesem Falle ist die Taumelvorrichtung 41 durch eine Vorspanneinrichtung 56 federnd vorgespannt. Auf eine solche Vorspanneinrichtung 56 kann alternativ aber auch verzichtet werden.

Des Weiteren umfasst das Taumelpumpenbasismodul 40 eine elastische Auflage 82. Die Auflage 82 ist in der Aufnahme 42 angeordnet, hier am Boden der Aufnahme 42. Toleranzen, die zwischen dem in die Aufnahme 42 eingelegten Pumpmodul 1 und dem Deckel 44 vorhanden sind, können durch die Elastizität der Auflage 82 ausgeglichen werden, wodurch der Deckel 44 hat ausgeführt werden kann. Damit wird das Pumpmodul 1 durch die Vorspanneinrichtung 56 fest gegen den Deckel 44 gedrückt und ist eindeutig positioniert. Die axiale Amplitude der Taumelvorrichtung 41, erzeugt durch die Vorspanneinrichtung 56, ist größer als die maximal zulässige axiale Beweglichkeit des Pumpmoduls 1, um ein sicheres Abquetschen der Membran 4 zu gewährleisten.

Die Anzahl der in den Ausführungsformen verwendeten Kugellager 73, 75, 76, 78 kann grundsätzlich variiert werden. Durch die Verwendung mehrerer Kugellager lässt sich die Bewegung exakter definieren bzw. stabilisieren, eine Reduzierung der verwendeten Kugellager ist vorteilhaft im Sinne eines kompakten und gewichtsparenden Aufbaus.

An Stelle einer in den Figuren 33 bis 37 gezeigten Taumelvorrichtung 41 mit Steg und Aussparung kann als eine Alternative auch eine Taumelvorrichtung 41 mit ebener Kontaktfläche, wie in der Fig. 9 gezeigt, verwendet werden. Die Pumpmodule 1 sind entsprechend mit einer wulstförmigen Membran 4 ausgebildet.

Taumelpumpenbasismodul 40 und Pumpenmodul 1 bilden gemeinsam ein Taumelpumpensystem zum Pumpen eines Fluids. Pumpenmodul 1 und Taumelpumpenbasismoduls 40 sind aufeinander abgestimmt, insbesondere die Taumelvorrichtung 41 des Taumelpumpenbasismoduls 40 mit dem Pumpkanal 5 des Pumpmoduls 1 und die Form des Pumpmoduls 1 mit der Form der Aufnahme 42 des Taumelpumpenbasismoduls 40. Ein derartiges Taumelpumpensystem stellt eine Pumpe mit definierten Pumpeigenschaften dar, bei der die durch die Förderung durch ein Fluid kontaminierten Bestandteile der Pumpe durch Wechseln des Pumpenmoduls 1 schnell und einfach ersetzt werden können. Das Pumpenmodul 1 ist kostengünstig und mit einem kompakten Aufbau als Einmalartikel ("Disposable") herstellbar. Das Pumpenmodul 1 kann insbesondere für verschiedene Anwendungen in einem Schlauchset oder einem Überleitsystem integriert sein.

Das Pumpmodul 1 kann zusätzlich ein Einwegeventil aufweisen, das einen ungewünschten Rückfluss des zu fördernden Fluids verhindert. Das Einwegeventil kann sowohl in Pumprichtung nach dem Pumpkanalausgang 7 oder vor dem Pumpkanaleingang 6 angeordnet sein. Insbesondere können auch mindestens zwei Einwegventile vorhanden sein, die sowohl nach dem Pumpkanalausgang 7 und vor dem Pumpkanaleingang 6 angeordnet sind. Vorzugsweise ist ein Einwegventil zwischen ersten Anschluss 15 und Pumpkanaleingang 6 und/oder zwischen zweiten Anschluss 16 und Pumpkanalausgang 7 angeordnet. Das mindestens eine Einwegeventil kann beispielsweise als flexible Membranklappe ausgebildet sein. Solche sind dem Fachmann hinlänglich bekannt. Das Einwegventil wird vorzugsweise durch das Basisteil 2 und/oder das Deckelteil 3 und einer flexiblen Membran gebildet.

Das erfindungsgemäße Pumpmodul 1 kann preiswert und robust hergestellt werden. Durch die Ausbildung des Pumpkanals 23 zumindest aus Membran 4 und Basisteil 2 lässt sich ein Pumpkanal 23 mit definierten und reproduzierbaren Abmessungen herstellen. Durch die periodisch umlaufende Membranverformung kann eine hohe Genauigkeit in der Förderrate erreicht werden. Ein Betrieb eines erfindungsgemäßen Pumpenmoduls 1 mit einer Taumelvorrichtung 41 hat den Vorteil, dass die mechanische Belastung der Membran 4 minimiert wird, weil kein rollender oder gleitender Kontakt mit der die Membran 4 verformenden Körper besteht. Dadurch, dass das Pumpmodul 1 kostengünstig und reproduzierbar herstellbar ist, eignet sich das erfindungsgemäße Pumpmodul 1 als Einwegartikel ("Disposable"), der lediglich zum einmaligen Gebrauch vorgesehen ist.

Die hier beschriebenen Ausführungsformen eines Pumpmoduls 1, eines Taumelpumpenbasismoduls 40 und eines Taumelpumpensystems eignen sich insbesondere für die Anwendung auf dem medizinischen Gebiet. Bevorzugte Anwendungen dieser Vorrichtungen sind die Anwendung als enterale Pumpen zum Pumpen von beispielsweise Ernährungslösungen oder als Infusionspumpen zum intravenösen Infundieren von Medikamenten. Andere Anwendungen sind ebenfalls denkbar.

## Patentansprüche

1. Pumpenmodul (1) insbesondere für eine Taumelpumpe, umfassend einen linienförmigen, zumindest abschnittsweise gekrümmten Pumpkanal (5), einen Pumpkanaleingang (6) und einen Pumpkanalausgang (7), wobei der Pumpkanaleingang (6) und der Pumpkanalausgang (7) mit dem Pumpkanal (5) zum Zuführen und Abführen eines Fluids in den Pumpkanal (5) verbunden ist, so dass durch eine periodisch umlaufende Verformung des Pumpkanals (5) ein Fluid durch den Pumpkanal (5) vom Pumpkanaleingang (6) zum Pumpkanalausgang (7) pumpbar ist, wobei der Pumpkanal (5) in dem Abschnitt zwischen Pumpkanaleingang (6) und Pumpkanalausgang (7) abweichend von einer reinen Kreisbogenform ausgebildet ist.

2. Pumpenmodul (1) nach Anspruch 2, wobei der Pumpkanal (5) in dem Abschnitt zwischen Pumpkanaleingang (6) und Pumpkanalausgang (7) einen spiralförmigen Abschnitt aufweist.

3. Pumpenmodul (1) nach Anspruch 1, wobei der Pumpkanal (5) in dem Abschnitt zwischen Pumpkanaleingang (6) und Pumpkanalausgang (7) einen ersten Pumpkanalbereich umfasst, der bogenförmig, vorzugsweise kreisbogenförmig, in einem ersten radialen Abstand zu einem Zentrum angeordnet ist, und einen zweiten Pumpkanalbereich umfasst der bogenförmig, vorzugsweise kreisbogenförmig, in einem, sich vom ersten radialen Abstand unterscheidenden zweiten radialen Abstand zum Zentrum angeordnet ist.

4. Pumpenmodul (1) nach Anspruch 1, wobei der Pumpkanal (5) in dem Abschnitt zwischen Pumpkanaleingang (6) und Pumpkanalausgang (7) sich in einen ersten Pumpkanalabschnitt und einen zweiten Pumpkanalabschnitt aufspaltet.

5. Pumpenmodul (1) nach Anspruch 2 bis 4, wobei der Pumpkanal (5) in dem Abschnitt zwischen Pumpkanaleingang (6) und Pumpkanalausgang (7) sich überlappt.

6. Pumpenmodul (1) nach einem der Ansprüche 1 bis 5, umfassend ein Basisteil (2) und eine elastisch verformbare Membran (4), wobei Basisteil (2) und Membran (4) den Pumpkanal (5) ausbilden, und das Basisteil (2) den Pumpkanaleingang (6) und den Pumpkanalausgang (7) umfasst.

7. Pumpenmodul (1) nach Anspruch 6, zusätzlich umfassend ein Deckelteil (3), wobei die Membran (4) mit dem Deckelteil (3) verbunden ist, und das Deckelteil (3) mit dem Basisteil (2) verbunden ist.

8. Pumpenmodul (1) nach einem der Ansprüche 6 bis 7, wobei das Basisteil (2), Membran (4) und/oder gegebenenfalls das Deckelteil (3) jeweils einteilig, vorzugsweise einstückig sind, und vorzugsweise aus einem Kunststoff bestehen, besonders bevorzugt Basisteil (2) und Deckelteil (3) aus demselben Kunststoff bestehen.

9. Taumelpumpenbasismodul (40), umfassend einen Taumelpumpenantrieb (43) mit einer Taumelvorrichtung (41), wobei die Taumelvorrichtung (41) einen in eine Taumelbewegung versetzbaren, linienförmigen und zumindest abschnittsweise gekrümmten Steg (46) zum taumelnden Verformen eines Schlauches (45) oder einer Membran (4) aufweist, wobei der Steg (46) abweichend von einer reinen Kreisbogenform ausgebildet ist.

10. Taumelpumpenbasismodul (40) nach Anspruch 10, wobei der Steg (46) einen kreisförmigen Abschnitt (47), einen ersten geraden Abschnitt (48) und einen zweiten geraden Abschnitt (49) aufweist, wobei der kreisförmige Abschnitt (47) zwischen dem ersten geraden Abschnitt (48) und dem zweiten geraden Abschnitt (49) angeordnet ist.

11. Taumelpumpenbasismodul (40) nach Anspruch 10, wobei der Steg (46) einen spiralförmigen Abschnitt (50) aufweist.

12. Taumelpumpenbasismodul (40) nach Anspruch 10, wobei der Steg (46) in einen ersten Abschnitt umfasst, der bogenförmig, vorzugsweise kreisbogenförmig, in einem ersten radialen Abstand zu einem Zentrum angeordnet ist, und einen zweiten Abschnitt umfasst, der bogenförmig, vorzugsweise kreisbogenförmig, in einem, sich vom ersten radialen Abstand unterscheidenden zweiten radialen Abstand zum Zentrum ausgebildet ist.

13. Taumelpumpenbasismodul (40) nach Anspruch 11 oder 12, wobei sich Abschnitte des Stegs (46) überlappen.

14. Taumelpumpenbasismodul (40) nach einem der Ansprüche 10 bis 13, wobei der Steg (46) mit einer konstanten oder variierenden Steghöhe ausgebildet ist.

15. Taumelpumpenbasismodul (40) nach Anspruch 11 oder 12, wobei der Steg (46) in einem ersten Abschnitt des spiralförmigen oder bogenförmigen Abschnitts eine größere Höhe aufweist als in einem zweiten, von Zentrum der Spirale entfernter liegenden zweiten Abschnitt des spiralförmigen oder bogenförmigen Abschnitts.

16. Taumelpumpensystem, umfassend eine Pumpenmodul (1) nach einem der Ansprüche 1 bis 8 und ein Taumelpumpenbasismodul (40) nach einem der Ansprüche 9 bis 15, wobei der Steg (46) der Taumelvorrichtung (41) des Taumelpumpenbasismoduls (40) in seinem Verlauf zumindest abschnittsweise entsprechend dem Abschnitt des Pumpkanals zwischen Pumpkanaleingang (6) und Pumpkanalausgang (7) ausgebildet ist.
